# EUROPÄISCHE PATENTSCHRIFT

(11) **EP 1 751 116 B1**
(45) Veröffentlichungstag und Bekanntmachung des Hinweises auf die Patenterteilung: **31.07.2013**
(21) Anmeldenummer: 05750829.3
(22) Anmeldetag: 03.06.2005
(51) Int. Cl.: C07D 239/00

(54) **Pyrimidinverbindungen und ihre Verwendung zur Behandlung von Erkrankungen des zentralen Nervensystems**
Pyrimidine compounds and use thereof in the treatment of central nervous system disorders
Composés de pyrimidine et leur utilisation dans le traitement d' affections liées au système nerveux central

(30) Priorität: 04.06.2004 DE 102004027358
(43) Veröffentlichungstag der Anmeldung: 14.02.2007
(60) Teilanmeldung: 11167864.5
(73) Patentinhaber: Abbott GmbH & Co. KG, 65205 Wiesbaden (DE)
(72) Erfinder: GENESTE, Hervé, 67141 Neuhofen (DE); HAUPT, Andreas, 68723 Schwetzingen (DE); BRAJE, Wilfried, 68163 Mannheim (DE); LUBISCH, Wilfried, 69118 Heidelberg (DE); STEINER, Gerd, 67281 Kirchheim (DE)
(74) Vertreter: Reitstötter - Kinzebach
(86) Internationale Anmeldenummer: PCT/EP2005/006000
(87) Internationale Veröffentlichungsnummer: WO 2005/118558

(56) Entgegenhaltungen:
- WO-A-97/25324
- A. ORJALES ET AL.: "New 3-benzoisothiazolyl and 3-benzoisoxazolylpiperazine derivatives with atypical antipsychotic binding profile" EUROPEAN JOURNAL OF MEDICINAL CHEMISTRY., Bd. 37, 2002, Seiten 721-730, XP004383155 EDITIONS SCIENTIFIQUE ELSEVIER; PARIS, FR

## Beschreibung

Die vorliegende Erfindung betrifft neue Pyrimidin-Verbindungen. Diese Verbindungen besitzen wertvolle therapeutische Eigenschaften und sind insbesondere zur Behandlung von Erkrankungen geeignet, die auf die Modulation des Dopamin-D₃-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Eine mögliche Behandlung dieser und anderer Erkrankungen basiert auf der Gabe von Substanzen, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren. In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514, J.N. Joyce, Pharmacology and Therapeutics 2001, 90, S. 231-59 "The Dopamine D3 Receptor as a Therapeutic Target for Antipsychotic and Antiparkinsonian Drugs").

Mittlerweile teilt man die Dopamin-Rezeptoren in zwei Familien ein: Einerseits die D₂-Gruppe, bestehend aus D₂-, D₃- und D₄-Rezeptoren, andererseits die D₁-Gruppe bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren, vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D₃ Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Pyrimidinverbindungen mit Dopamin-D₃-Rezeptoraffinität wurden verschiedentlich im Stand der Technik beschrieben, z.B. in WO 96/02519, WO 96/02520, WO 96/02249, WO 96/02246, WO 99/02503, WO 00/42036, WO 00/42037, WO 00/42038. Diese Verbindungen besitzen teilweise hohe Affinitäten zum Dopamin-D₃-Rezeptor. Sie werden daher zur Behandlung von Erkrankungen des zentralen Nervensystems vorgeschlagen.

Es besteht jedoch grundsätzlich ein Bedarf, weitere Verbindungen mit Dopamin-D₃-Rezeptoraffinität bereitzustellen, sei es, um das pharmakologische Bindungsprofil zu verbessern, oder weil die Verbindungen Standes der Technik unerwünschte Nebenwirkungen hervorrufen, eine schlechte Cerebralverfügbarkeit aufweisen oder nur eine geringe Bioverfügbarkeit besitzen. Der Erfindung liegt daher die Aufgabe zu Grunde, weitere Verbindungen zur Verfügung zu stellen, die als selektive Dopamin-D₃-Rezeptor-Liganden wirken.

Diese Aufgabe wird gelöst durch Verbindungen, welche der allgemeinen Formel I 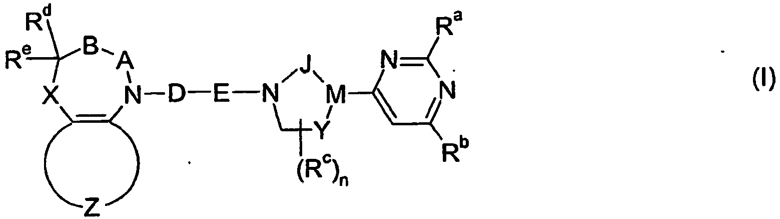 gehorchen, worin
- A: für eine Gruppe C=W oder CR^{f}R^{g} steht;
- B: für eine chemische Bindung oder eine Gruppe CR^{h}Rⁱ steht;
- X: für O, S, eine Gruppe N-R^{k} oder eine Gruppe CR^{m}Rⁿ steht;
- D: für C=O oder eine chemische Bindung steht;
- E: für eine lineare oder verzweigte 2 bis 10 gliedrige Alkylenkette steht, die als Kettenglieder 1 oder 2 nicht benachbarte Heteroatomgruppe(n) K aufweisen kann, die ausgewählt ist unter O, S, S(O), S(O)₂ und N-R^{P}, und die eine Carbonylgruppe und/oder eine Cycloalkandiyl-Gruppe umfassen kann und/oder eine Doppel- oder Dreifachbindung aufweisen kann;
- W: Sauerstoff oder Schwefel bedeutet;
- Z: gemeinsam mit den C-Atomen, an die es gebunden ist, für einen Phenylring oder einen Cyclohexenring steht, wobei Cyclohexenring eine Carbonylgruppe als Ringglied aufweisen kann und wobei der Phenylring 1, 2, 3 oder 4 Substituenten R aufweisen kann, die ausgewählt sind unter gegebenenfalls substituiertem C₁-C₆-Alkyl, CN, OR¹, NR²R³, NO₂, SR⁴, SO₂R⁴, SO₂NR²R³, CONR²R³, COOR⁵, COR⁶, C₁-C₄-Halogen-alkoxy, C₂-C₆-Alkenyl, C₂-C₆-Alkinyl, C₂-C₆-Alkenyloxy, C₂-C₆-Alkinyloxy, C₃-C₆-Cycloalkyl, C₃-C₆-Cycloalkyloxy und Halogen, und/oder 2 Substituenten R gemeinsam eine Kette X'-Alk'-X" bilden können, worin X' und X" unabhängig voneinander für O oder S stehen und Alk' C₁-C₄-Alkandiyl bedeutet, das gegebenenfalls 1, 2, 3 oder 4 Alkylgruppen oder Halogenatome als Substituenten aufweist;
- J: für CH₂-CH₂ steht;
- M: für N steht;
- Y: für CH₂ oder CH₂-CH₂ steht;
- n: 0 oder 1 ist;
- R^{a}, R^{b}: unabhängig voneinander für C₁-C₆-Alkyl, C₁-C₆-Halogenalkyl, C₂-C₆-Alkenyl, C₃-C₆-Cycloalkyl, C₁-C₆-Alkoxy-C₁-C₄-alkyl oder C₃-C₆-Cycloalkyl-C₁-C₄-alkyl stehen;
- R^{c}: für C₁-C₄-Alkyl steht;
- R^{d}, R^{e}: unabhängig voneinander ausgewählt sind unter Wasserstoff, Halogen, gegebenenfalls substituiertem C₁-C₆-Alkyl, C₃-C₆-Cycloalkyl, C₂-C₆-Alkenyl, C₂-C₆-Alkinyl, C₁-C₆-Alkoxy, C₃-C₆-Cycloalkyloxy, C₃-C₆-Cycloalkyl-C₁-C₄-alkyloxy und gegebenenfalls substituiertem Phenyl, wobei CR^{d}R^{e} gemeinsam für C=O stehen können und die Reste R^{d}, R^{e} gemeinsam eine Kette X'-Alk-X" bilden können, worin X und X" unabhängig voneinander für O oder S stehen und Alk C₂-C₄-Alkandiyl bedeutet, das gegebenenfalls 1, 2 3 oder 4 Alkylgruppen oder Halogenatome als Substituenten aufweist;
- R^{f}, R⁹: unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆-Cycloalkyloxy, C₃-C₆-cycloalkyl-C₁-C₄-alkyloxy oder C₃-C₆-Cycloalkyl bedeuten oder die Reste R^{f}, R^{g} gemeinsam eine Kette X'-Alk-X" bilden können, worin Alk, X' und X" die zuvor genannten Bedeutungen aufweisen;
- R^{h}, Rⁱ: unabhängig voneinander Wasserstoff, Halogen, gegebenenfalls substituiertes C₁-C₆-A]kyl, C₁-C₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten;
- R^{k}, R^{p}: unabhängig voneinander für Wasserstoff, C₁-C₄-Alkyl, C₁-C₆-Alkoxy, C₁-C₆-Alkoxyalkyl, C₁-C₆-Alkylcarbonyl, C₁-C₆-Alkoxycarbonyl, Phenylalkyl, Phenylcarbonyl, Phenoxycarbonyl, wobei Phenyl in den drei letzgenannten Gruppen 1, 2 oder 3 Substituenten tragen kann, die ausgewählt sind unter C₁-C₄-Alkyl, C₁-C₄-Alkoxy, NR²R³, CN, C₁-C₂-Fluoralkyl und Halogen;
- R^{m}, Rⁿ: unabhängig voneinander Wasserstoff, Halogen, gegebenenfalls substituiertes C₁-C₆-Alkyl, C₁-C-₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten; und
wobei, wenn X für CR^{m}Rⁿ oder N-R^{k} steht, einer der Reste R^{d} oder R^{e} gemeinsam mit einem der Reste R^{m}, Rⁿ oder R^{k} auch eine π-Bindung bedeuten können;
- R¹, R², R³, R⁴, R⁵ und R⁶: unabhängig voneinander für H, gegebenenfalls substituiertes C₁-C₆-Alkyl oder gegebenenfalls substituiertes Phenyl, stehen, wobei R³ auch eine Gruppe COR⁷ bedeuten kann, wobei R⁷ für Wasserstoff, gegebenenfalls substituiertes C₁-C₄-Alkyl oder gegebenenfalls substituiertes Phenyl steht, wobei R² mit R³ auch gemeinsam mit dem Stickstoffatom, an das sie gebunden sind, einen 5- oder 6-gliedrigen, gesättigten oder ungesättigten Heterocyclus bilden können, der ein weiteres Heteroatom, ausgewählt unter O, S und NR⁸ als Ringglied aufweisen kann, wobei R⁸ für Wasserstoff oder C₁-C₄-Alkyl steht, und
die physiologisch akzeptablen Säureadditionssalze dieser Verbindungen.

Gegenstand der vorliegenden Erfindung sind daher die Verbindungen der allgemeinen Formel I sowie die physiologisch verträglichen Säureadditionssalze der Verbindungen I.

Gegenstand der vorliegenden Erfindung ist auch die Verwendung der Verbindungen der allgemeinen Formel I, sowie der physiologisch verträglichen Säureadditionssalze der Verbindungen I zur Herstellung eines pharmazeutischen Mittels zur Behandlung von Erkrankungen, die auf die Beeinflussung durch Dopamin-D₃-Rezeptorantagonisten bzw. -agonisten ansprechen.

Zu den Erkrankungen, die auf die Beeinflussung durch Dopamin-D₃-Rezeptorantagonisten bzw. -agonisten ansprechen zählen insbesondere Störungen und Erkrankungen des zentralen Nervensystems, insbesondere affektiven Störungen, neurotische Störungen, Belastungs- und somatoformen Störungen und Psychosen, speziell Schizophrenie und Depression und weiterhin Nierenfunktionsstörungen, insbesondere Nierenfunktionsstörungen, die durch Diabetes mellitus hervorgerufen werden (siehe WO 00/67847).

Man verwendet zur Behandlung der vorstehend genannten Indikationen wenigstens eine Verbindung der allgemeinen Formel I oder ein physiologisch verträgliches Säureadditionssalz einer Verbindung I. Sofern die Verbindungen der Formel I ein oder mehrere Asymmetriezentren aufweisen oder Tautomere bilden, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Ebenfalls brauchbar sind physiologisch verträgliche Salze der Verbindungen der Formel I, vor allem Säureadditionssalze mit physiologisch verträglichen Säuren. Als physiologisch verträgliche organische und anorganische Säuren kommen beispielsweise Chlorwasserstoffsäure, Bromwasserstoffsäure, Phosphorsäure, Schwefelsäure, C₁-C₄-Alkylsulfonsäuren wie Methansulfonsäure, aromatische Sulfonsäuren wie Benzolsulfonsäure und Toluolsulfonsäure, Oxalsäure, Maleinsäure, Fumarsäure, Milchsäure, Weinsäure, Adipinsäure und Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Halogen steht hier und im folgenden für Fluor, Chlor, Brom oder lod.

Cₙ-Cₘ-Alkyl (auch in Resten wie Alkoxy, Alkoxyalkyl, Alkylthio, Alkylamino, Dialkylamino, Alkylcarbonyl etc.) bedeutet eine geradkettige oder verzweigte Alkylgruppe mit n bis m Kohlenstoffatomen, z.B. 1 bis 6 und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl, n-Hexyl und dergleichen.

Der Ausdruck "gegebenenfalls substituiertes Cₙ-Cₘ-Alkyl" steht für einen Alkylrest mit n bis m C-Atomen, der teilweise oder vollständig durch Halogen, insbesondere durch Chlor oder Fluor substituiert sein kann und der einen oder mehrere, z.B. 1, 2 oder 3 von Halogen verschiedene Substituenten tragen kann, die ausgewählt sind unter CN, C₃-C₇-Cycloalkyl, C₃-C₇-Heterocycloalkyl, gegebenenfalls substituiertem Phenyl, OR¹¹, COOR¹¹, NR¹²R¹³, SO₂NR¹²R¹³, CONR¹²R¹³, O-CONR¹²R¹³, S-R¹⁴, SOR¹⁵, SO₂R¹⁵, OCOR¹⁶ und COR¹⁶. Hierin hat R" die für R¹ angegebene Bedeutung, R¹² die für R² angegebene Bedeutung, R¹³ die für R³ angegebene Bedeutung, R¹⁴ die für R⁴ angegebene Bedeutung, R¹⁵ die für R⁵ angegebene Bedeutung und R¹⁶ die für R⁶ angegebene Bedeutung. Insbesondere stehen R" - R¹⁶ für Wasserstoff, C₁-C₄-Alkyl, C₁-C₄-Halogenalkyl, C₃-C₇-Cycloalkyl, gegebenenfalls substituiertes Benzyl oder gegebenenfalls substituiertes Phenyl.

Bevorzugte Substituenten an Alkyl sind unter OH, C₁-C₄-Alkoxy, Halogen, C₃-C₇-Cycloalkyl und gegebenenfalls substituiertem Phenyl ausgewählt. Im Falle von OH, C₁-C₄-Alkoxy, C₃-C₇-Cycloalkyl und Phenyl ist es insbesondere nur ein Substituent. Derartige Reste werden im Folgenden auch als C₁-C₄-Alkoxy-C₁-C₆-alkyl wie Methoxymethyl, 1-oder 2-Methoxyethyl, 1-Methoxy-1-methylethyl oder 2-Methoxy-1-methylethyl, 1-, 2-oder 3-Methoxypropyl, Ethoxymethyl, 1- oder 2-Ethoxyethyl, Hydroxy-C₁-C₆-alkyl, 1-Hydroxymethyl, 1- oder 2-Hydroxyethyl, 1-Hydroxy-1-methylethyl, 1-, 2- oder 3-Hydroxypropyl etc., C₃-C₆-Cycloalkyl-C₁-C₆-alkyl wie Cyclopropylmethyl, Cyclohexylmethyl oder Phenyl-C₁-C₆-alkyl bezeichnet. Im Falle von Halogen-Substituenten werden diese Reste auch als Halogenalkyl bezeichnet.

C₁-C₆-Halogenalkyl (auch in Resten wie C₁-C₆-Halogenalkoxy) steht für eine Alkylgruppe mit 1 bis 6 und insbesondere 1 bis 4 C-Atomen, wie oben definiert, in der alle oder ein Teil, z.B. 1, 2, 3, 4 oder 5 der Wasserstoffatome durch Halogenatome, insbesondere durch Chlor oder Fluor ersetzt sind. Bevorzugtes Halogenalkyl ist C₁-C₂-Fluoralkyl oder C₁-C₂-fluorchloralkyl, insbesondere CF₃, CHF₂, CF₂Cl, CH₂F, CH₂CF₃.

C₃-C₁₀-Cycloalkyl, auch in Resten wie Cycloalkylalkyl, Cycloalkylcarbonyl und Cycloalkylcarbonylalkyl steht für einen cycloaliphatischen Rest mit 3 bis 10 und vorzugsweise 3 bis 6 C-Atomen wie Cyclopropyl, Cyclobutyl, Cyclopentyl, Cyclohexyl, Cycloheptyl und Cyclooctyl.

C₃-C₁₀-Heterocycloalkyl, auch in Resten wie Heterocycloalkylalkyl, Heterocycloalkylcarbonyl und Heterocycloalkylcarbonylalkyl steht für einen gesättigten heterocyclischen Rest mit Ringgliedem, wobei 1, 2 oder 3 Ringglieder ein Heteroatom ausgewählt unter N, O und S sind wie Oxiranyl, Oxetanyl, Aziranyl, Azetanyl, Tetrahydrofurfuryl, Tetrahydrothienyl, Pyrrolidinyl, Pyrazolinyl, Imidazolinyl, Piperidinyl, Piperazinyl oder Morpholinyl.

C₄-C₁₀-Bicycloalkyl steht für einen bicycloaliphatischen Rest mit 4 bis 10 C-Atomen wie in Bicyclo[2.1.0]pentyl, Bicyclo[2.1.1]hexyl, Bicyclo[3.1.0]hexyl, Bicyclo[2.2.1]heptanyl, Bicyclo[3.2.0]heptanyl, Bicydo[2.2.2]octanyl, Bicydo[3.2.1]octanyl, Bicyclo[3.3.1]nonyl und Bicyclo[4.4.0]decyl.

C₆-C₁₀-Tricycloalkyl steht für einen tricycloaliphatischen Rest mit 6 bis 10 C-Atomen wie in Adamantyl.

C₂-C₆-Alkenyl steht für einen einfach ungesättigten linearen oder verzweigten Kohlenwasserstoffrest mit 2, 3, 4, 5 oder 6 C-Atomen, z.B. für Vinyl, Allyl (2-Propen-1-yl), 1-Propen-1-yl, 2-Propen-2-yl, Methallyl (2-Methylprop-2-en-1-yl) und dergleichen. C₃-C₄-Alkenyl steht insbesondere für Allyl, 1-Methylprop-2-en-1-yl, 2-Buten-1-yl, 3-Buten-1-yl oder Methallyl.

C₂-C₆-Halogenalkenyl steht für eine Alkenylgruppe wie oben definiert, in der alle oder ein Teil, z.B. 1, 2, 3, 4 oder 5 der Wasserstoffatome durch Halogenatome, insbesondere durch Chlor oder Fluor ersetzt sind.

C₂-C₆-Alkinyl steht für einen Kohlenwasserstoffrest mit 2, 3, 4, 5 oder 6 C-Atomen, der eine Dreifachbindung aufweist, z.B. für Propargyl (2-Propin-1-yl), 1-Methylprop-2-in-1-yl, 2-Butin-1-yl, 3-Butin-1-yl, 2-Pentin-1-yl, 1-Pentin-3-yl etc.

C₂-C₆-Halogenalkinyl steht für eine Alkinylgruppe wie oben definiert, in der alle oder ein Teil, z.B. 1, 2, 3, 4 oder 5 der Wasserstoffatome durch Halogenatome, insbesondere durch Chlor oder Fluor ersetzt sind.

Phenyl-C₁-C₄-alkyl steht für eine C₁-C₄-Alkylrest, wie oben definiert, in dem ein Wasserstoffatom durch einen Phenylrest ersetzt ist wie in Benzyl oder 2-Phenylethyl.

Gegebenenfalls substituiertes Phenyl steht für Phenyl, dass gegebenenfalls einen oder mehrere, z.B. 1, 2 oder 3 der nachfolgenden Substituenten aufweist: Halogen, Nitro, Cyano, gegebenenfalls substituiertes C₁-C₄-Alkyl, C₂-C₆-Alkenyl, C₂-C₆-Alkinyl, C₃-C₆-Cycloalkyl, C₁-C₄-Halogenalkyl, C1-C₄-Alkoxy, C₁-C₄-Alkylsulfonylamino, OR²¹, COOR²¹, NR²²R²³, SO₂NR²²R²³, CONR²²R²³, O-CONR²²R²³, S-R²⁴, SOR²⁵, SO₂R²⁵, OCOR²⁶ und COR²⁶. Beispiele für geeignete Substituenten an Phenyl sind insbesondere Halogen, C₁-C₄-Alkyl, C₂-C₆-Alkenyl, C₂-C₆-Alkinyl, C₃-C₆-Cycloalkyl, C₁-C₄-Halogenalkyl, C₁-C₄-Alkoxy, C₁-C₄-Alkoxy-C₁-C₄-alkyl, Hydroxy-C₁-C₄-alkyl, Hydroxy, Nitro, NH₂, Cyano, COOH, C₁-C₄-Alkoxycarbonyl, C₁-C₄-Alkylcarbonyl, C₁-C₄-Alkylamino, Di-(C₁-C₄-alkyl)amino, C₁-C₄-Alkylsulfonyl, C₁-C₄-Alkylsulfonylamino und/oder C₁-C₄-Alkylaminosulfonyl. Hierin hat R²¹ die für R¹ angegebene Bedeutung, R²² die für R² angegebene Bedeutung, R²³ die für R³ angegebene Bedeutung, R²⁴ die für R⁴ angegebene Bedeutung, R²⁵ die für R⁵ angegebene Bedeutung und R²⁶ die für R⁶ angegebene Bedeutung. Insbesondere stehen R²¹ - R²⁶ für Wasserstoff. C₁-C₄-Alkyl, C₁-C₄-Halogenalkyl, C₃-C₇-Cycloalkyl, gegebenenfalls substituiertes Benzyl oder gegebenenfalls substituiertes Phenyl.

Der Begriff "Alkylen" umfasst grundsätzlich geradkettige oder verzweigte Reste mit vorzugsweise mit 2 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. C₀-Alkylen steht für eine Einfachbindung, C₁-Alkylen für Methylen und C₂-Alkylen für 1,1-Ethylen oder 1,2-Ethylen.

Der Begriff 3 bis 8 gliedriges Heterocyclyl umfasst gesättigte (= Heterocycloalkyl), teilweise ungesättigte heterocyclische Reste und aromatische Heterocyclen (Heteroaryl) der Ringgröße 3, 4, 5, 6, 7 und 8, insbesondere der Ringgröße 5 oder 6, die 1, 2 oder 3 Heteroatome als Ringglieder aufweisen. Die Heteroatome sind dabei ausgewählt unter O, S und N.

Beispiele für gesättigtes 3- bis 8-gliedriges Heterocyclyl sind Oxiranyl, Oxetanyl, Aziranyl, Piperidinyl, Piperazinyl, Morpholinyl, Pyrrolidinyl, Oxazolidinyl, Tetrahydrofuryl, Dioxolanyl, Dioxanyl, Hexahydroazepinyl, Hexyhydrooxepinyl, und Hexahydrothiepinyl.

Beispiele für teilweise ungesättigtes 3- bis 8 gliedriges Heterocyclyl sind Di- und Tetrahydropyridinyl, Pyrrolinyl, Oxazolinyl, Dihydrofuryl, Tetrahydroazepinyl, Tetrahydrooxepinyl, und Tetrahydrothiepinyl.

Beispiele für 5- gliedrige heteroaromatische Reste (= 5-gliedriges Heteroaryl) sind solche mit 1, 2, 3 oder 4 Heteroatomen als Ringglieder, die unabhängig voneinander ausgewählt sind unter O, N und S, z.B. Pyrrol, Thiophen, Furan, Oxazol, Isoxazol, Thiazol, Isothiazol, Imidazol, 1,2,3-Thiadiazol, 1,2,4-Thiadiazol, 1,3,4-Thiadiazol, 1,2,3-Triazol, 1,2,4-Triazol, 1,3,4-Triazol, Tetrazol. Beispiele für 6-gliedrige heteroaromatische Reste (= 6-gliedriges Heteroaryl), die 1 oder 2 Stickstoffatome als Ringglieder aufweisen, sind vor allem 2-, 3- oder 4-Pyridinyl, 2-, 4- oder 5-Pyrimidinyl, 2- oder 3-Pyrazinyl sowie 3-oder 4-Pyridazinyl.

In der Gruppe E befinden sich die beiden Bindungstellen der Alkylenkette in der Regel nicht an dem gleichen C-Atom sondern bilden, gegebenenfalls zusammen mit der Heteroatomgruppe K eine wenigstens zwei-, vorzugsweise wenigstens drei-, und insbesondere wenigstens viergliedrige Kette, die, wenn D für eine Bindung steht, das mit A verknüpfte Stickstoffatom von dem Stickstoffatom des Stickstoffheterocyclus, oder wenn D für eine Carbonylgruppe steht, die Carbonylgruppe von dem Stickstoffatom des Stickstoffheterocyclus, an das E gebunden ist, durch mindestens 3, vorzugsweise durch mindestens 4 und insbesondere durch mindestens 5 Bindungen voneinander trennt. Wenn E keine Heteroatomgruppe K aufweist, umfasst E vorzugsweise 4 bis 10 Kohlenstoffatome, insbesondere 4 bis 8 Kohlenstoffatome und besonders bevorzugt 4 bis 6 Kohlenstoffatome als Kettenglieder. Wenn E eine Heteroatomgruppe K aufweist, umfasst E neben dieser Gruppen insbesondere 2 bis 8 Kohlenstoffatome und speziell 3 bis 5 Kohlenstoffatome als Kettenglieder. Außerdem können die gesättigten C-C-Bindungen in E durch ungesättigte Bindungen (Alkenylen; Alkinylen) ersetzt sein. So können sich geradkettige oder verzweigte ungesättigte Reste ergeben, deren Anzahl und Anordnung der Kohlenstotfatome derjenigen der zuvor genannten Alkylenreste entspricht, wobei jedoch eine oder mehrere Einfachbindungen durch entsprechende ungesättigte Doppel- bzw. Dreifachbindungen ersetzt sind. Außerdem kann E einen Cycloalkandiyl-Rest, vorzugsweise einen C₃-C₇-Cyloalkandlyl-Rest, Insbesondere einen C₄-C₇-Cyloalkan-1,2-, -1,3- oder 1,4-diyl-Rest umfassen, z.B. Cyclopropan-1,2-diyl, Cyclobutan-1,2- oder 1,3-diyl, Cyclopentan-1,2-oder-1,3-diyl, Cyclohexan-1,2-, 1,3-oder -1,4-diyl-Rest, oder einen Cycloheptan-1,2-, -1,3- oder 1,4-diyl-Rest. Dieser Cyloalkandiylrest ist Bestandteil der Kette E. Mit anderen Worten, ein Teil des Cycloalkandiyl-Restes bildet mit den übrigen Kettengliedern die Kette E, wobei hinsichtlich der Kettenlänge der kleinere Teil des Cycloalkandiyl-Restes maßgeblich ist.

Wenn die Alkylengruppe in E wenigstens ein Heteroatom, eine Heteroatomgruppe K umfasst, kann diese in der Alkylenkette an beliebiger Stelle angeordnet sein. Vorzugsweise ist das Heteroatom nicht an das Stickstoffatom gebunden. Vorzugsweise ist eine Carbonylgruppe an das zu A benachbarte Stickstoffatom gebunden.

Beispiele für geeignete Gruppen E sind:
(CH₂)ₖ mit k = 2, 3, 4, 5, 6, 7, 8, 9 oder 10, vorzugsweise 3, 4, 5 oder 6 und speziell 3 oder 4;
CH(CH₃)(CH₂), mit I = 1, 2, 3, 4, 5, 6, 7, 8 oder 9, insbesondere 2, 3, 4 oder 5 und speziell 2 oder 3;
CH₂CH(CH₃)(CH₂)ₖ. mit k' = 0, 1, 2, 3, 4, 5, 6, 7 oder 8, cis- und *trans*-CH₂-CH=CHCH₂, *cis-* und *trans*-CH₂-C(CH₃)=CH-CH₂, *cis-* und trans-CH₂CH₂CH=CHCH₂, *cis-* und *trans-*CH₂CH₂C(CH₃)=CHCH₂, CH₂C(=CH₂)CH₂, CH₂CH₂CH(CH₃)CH₂, 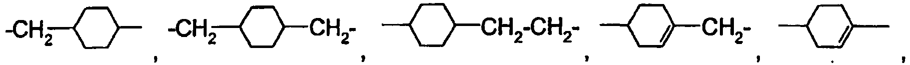 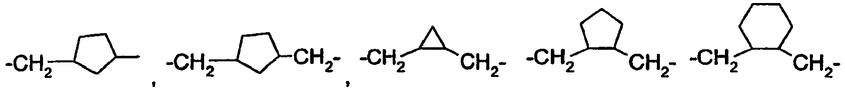
CH₂-O-CH₂-CH₂, CH₂-O-CH₂-CH₂-CH₂ und -CH₂-CH2-O-CH₂-CH₂-.

Im Hinblick auf die Verwendung der erfindungsgemäßen Verbindungen als Dopamin-D₃-Rezeptor-Liganden sind solche Verbindungen I besonders bevorzugt, worin E in Formel I für C₃-C₁₀-Alkylen, insbesondere für C₃-C₈-Alkylen und speziell für C₃-C₆-Alkylen steht, das eine Doppelbindung aufweisen kann. Insbesondere steht E für eine Gruppe (CH₂)ₖ, worin k die zuvor genannten Bedeutungen aufweist. Besonders bevorzugte Gruppe E sind außerdem *cis*- und *trans*-CH₂-CH=CH-CH₂, *cis-* und *trans*-CH₂-C(CH₃)=CH-CH₂, CHCH₃-CH₂-CH₂-CH₂ und CH₂-CHCH₃-CH₂-CH₂.

Sofern A für C=W steht, dann bedeutet D vorzugsweise eine chemische Bindung. W steht insbesondere für Sauerstoff. Wenn A für CR^{f}R^{g} steht, dann bedeutet D vorzugsweise eine Carbonylgruppe. R^{f} und R^{g} stehen unabhängig voneinander insbesondere für Wasserstoff oder C₁-C₄-Alkyl, speziell Methyl. Insbesondere steht wenigstens einer und speziell beide Reste R^{f} und R⁹ für Wasserstoff.

X in Formel I steht Insbesondere für O oder eine Gruppe CR^{m}Rⁿ, worin R^{m} und Rⁿ die zuvor genannten Bedeutungen aufweisen, insbesondere für Wasserstoff oder C₁-C₄-Alkyl, speziell Methyl, und besonders bevorzugt für Wasserstoff stehen. Besonders bevorzugt steht X für CH₂.

Eine bevorzugte Ausführungsform der Erfindung betrifft Verbindungen der allgemeinen Formel I, worin Z gemeinsam mit den C-Atomen, an die es gebunden ist, für einen Phenylring steht, der unsubstituiert ist oder 1, 2, 3 oder 4, insbesondere 1 oder 2 der zuvor genannten Substituenten R aufweisen kann. Insbesondere ist der Phenylring unsubstituiert oder weist einen der vorgenannten Substituenten R auf. Dieser ist vorzugsweise an das Kohlenstoffatom gebunden, das den Brückenkopfatomen benachbart ist und insbesondere an das Kohlenstoffatom, welches dem Brückenkopfatom benachbart ist, das an die Gruppe X gebunden ist. Bevorzugt sind auch solche Verbindungen, worin Z gemeinsam mit den C-Atomen, an die es gebunden ist einen gegebenenfalls substituierten Cyclohexenring bildet, der gegebenenfalls eine Carbonylgruppe als Ringglied aufweist, z.B. einen Cyclohex-2-enonring.

Bevorzugte Substituenten R an Z sind C₁-C₄-Alkyl, speziell Methyl, Ethyl, n-Propyl, n-Butyl und tert.-Butyl, OH, Halogen, insbesondere F, Cl oder Br, C₁-C₄-Alkoxy, speziell Methoxy, C₁-C₄-Alkylamino, Di-C₁-C₄-alkylamino, speziell N(CH₃)₂, C₁-C₄-Alkylcarbonyl, speziell Acetyl, C₁-C₄-Alkoxycarbonyl, speziell Methoxycarbonyl, C₁-C₄-Alkylsulfonyl, speziell Methylsulfonyl, C₁-C₄-Haloalkyl, speziell Trifluormethyl, C₁-C₄-Haloalkoxy, speziell Trifluormethoxy und Difluormethoxy, CN und C(O)NH₂, insbesondere C₁-C₄-Alkyl, OH, Halogen und C₁-C₄-Alkoxy, speziell OH, Methoxy, F, Cl, Br, CF₃ und CCF₃. Bevorzugt ist auch, wenn 2 an benachbarte C-Atome gebundene Gruppen R für O-Alk'-O stehen, worin Alk' für CH₂, CF₂, CH₂-CH₂ oder C(CH₃)₂ steht

Hinsichtlich ihrer Eigenschaft als selektive Dopamin-D₃-Rezeptorliganden steht in Verbindungen der allgemeinen Formel I M für ein Stickstoffatom. J steht für CH₂-CH₂. Y steht für CH₂ oder CH₂-CH₂. In einer besonders bevorzugten Ausführungsform steht J in Formel I für CH₂-CH₂ und Y für CH₂.

Hinsichtlich ihrer Eigenschaft als selektive Dopamin-D₃-Rezeptorliganden sind außerdem Verbindungen der allgemeinen Formel I bevorzugt, worin R^{a} und R^{b} unabhängig voneinander für C₁-C₆-Akyl, C₁-C₆-Halogenalkyl, C₂-C₆-Alkenyl, C₃-C₆-Cycloalkyl, C₁-C₆-Alkoxy-C₁-C₄-alkyl oder C₃-C₆-Cycloalkyl-C₁-C₄-alkyl stehen. R^{a} steht insbesondere für C₁-C₆-Alkyl, besonders bevorzugt für verzweigtes C₃-C₆-Alkyl und speziell für tert.-Butyl. R^{b} ist insbesondere ausgewählt ist unter C₁-C₆-Alkyl, C₃-C₆-Cycloalkyl und C₁-C₂-Fluoralkyl, besonders bevorzugt n-Propyl, Trifluormethyl, Cyclopropyl oder Cyclobutyl.

Eine bevorzugte Ausführungsform der Erfindung betrifft Verbindungen der allgemeinen Formel I, worin B eine chemische Bindung bedeutet.

In einer anderen bevorzugten Ausführungsform der Erfindung steht B für eine Gruppe CR^{h}Rⁱ, worin R^{h}, Rⁱ unabhängig voneinander insbesondere für Wasserstoff oder C₁-C₄-Alkyl, speziell Methyl, stehen. Insbesondere steht wenigstens einer und speziell beide Reste R^{h} und Rⁱ für Wasserstoff.

Die Variablen R^{d} und R^{e} stehen unabhängig voneinander insbesondere für Wasserstoff oder C₁-C₄-Alkyl, speziell Methyl und besonders bevorzugt beide für Wasserstoff.

Insbesondere steht die Gruppe der Formel 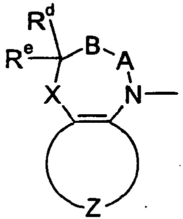 für einen der im folgenden angegebenen Reste a bis e: 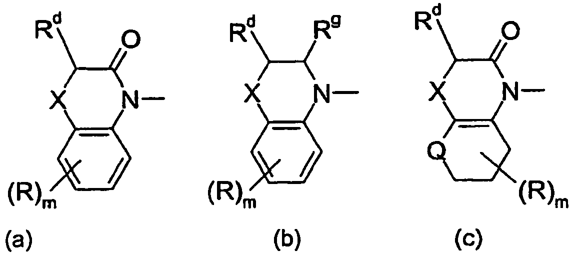 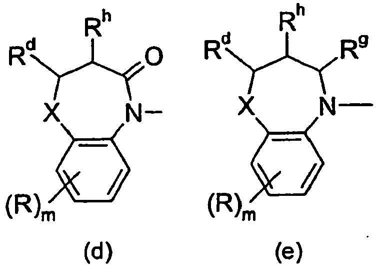 worin X, R, R^{d}, R^{g} und R^{h} die zuvor genannten Bedeutungen aufweisen, m = 0, 1, 2 oder 3, insbesondere m = 0 oder 1 ist in den Resten a, b, d und e und m = 0 ist in dem Rest c und Q für CH₂ oder eine Carbonylgruppe steht.

Dementsprechend sind bevorzugte Ausführungsformen der Erfindung die Verbindungen der im folgenden angegebenen allgemeinen Formel Ia bis Ie, 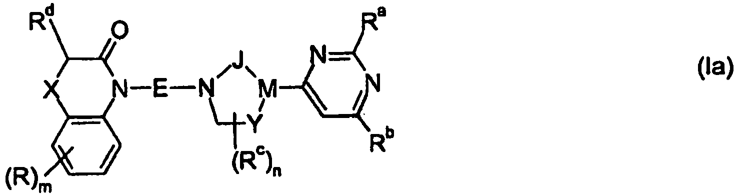 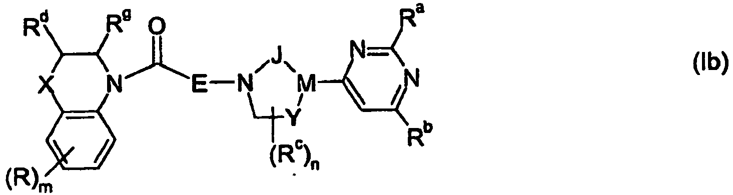 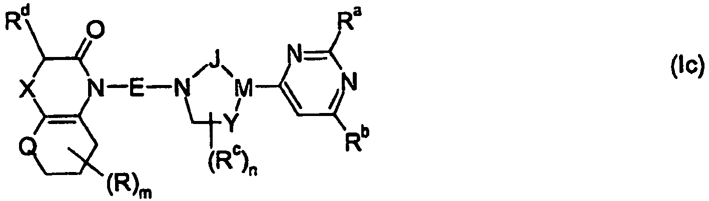 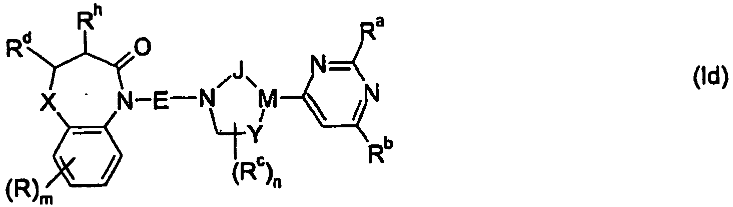 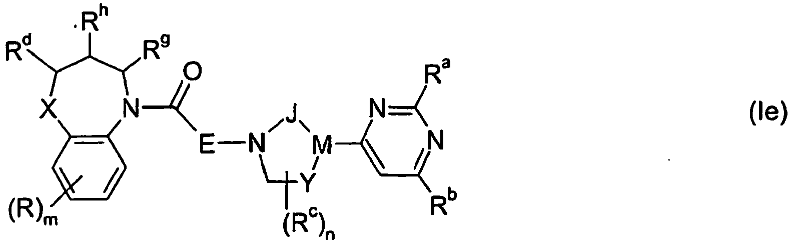 worin n, m Q, X, E, J, M, R, R^{a}, R^{b}, R^{c}, R^{d}, R^{g} und R^{h} die zuvor genannten Bedeutungen und insbesondere gemeinsam die als bevorzugt genannten Bedeutungen aufweisen.

Unter den Verbindungen Ia sind die Verbindungen der nachfolgenden Formeln Ia.1 bis Ia.8 besonders bevorzugt: 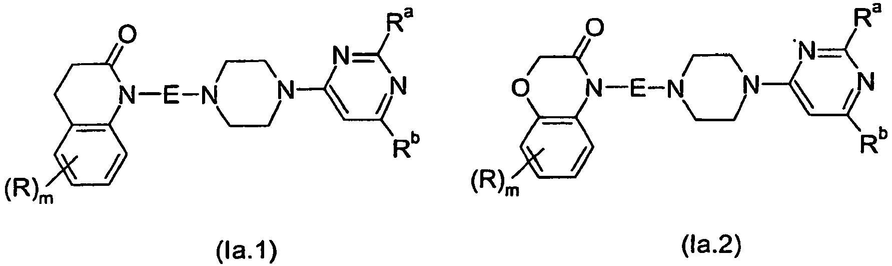 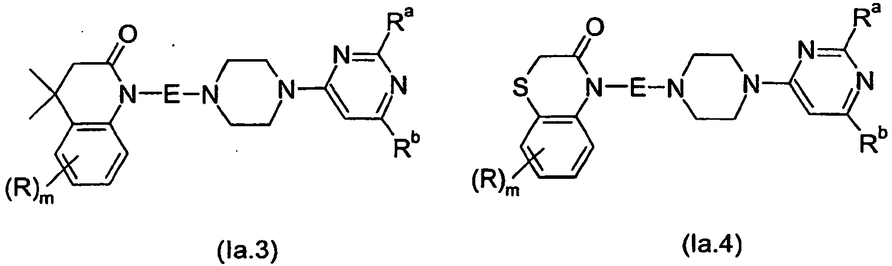 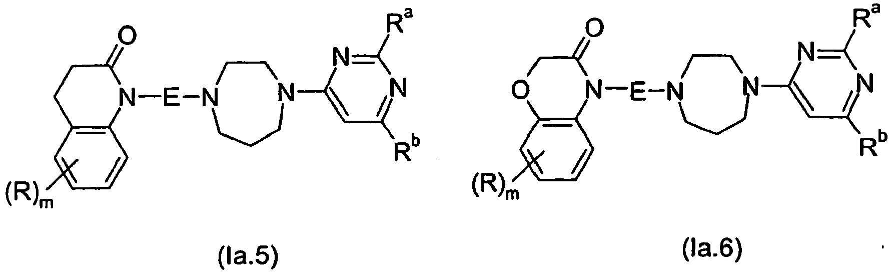 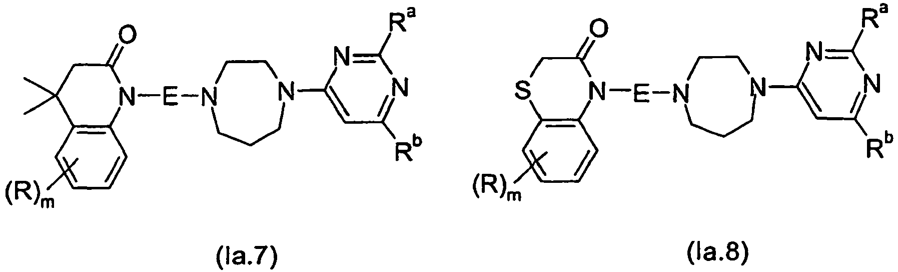 worin m, E, R, R^{a} und R^{b} die zuvor genannten Bedeutungen und insbesondere gemeinsam die als bevorzugt genannten Bedeutungen aufweisen.

Beispiele hierfür sind die Verbindungen der Formeln Ia.1, Ia.2, Ia.3, Ia.4, Ia.5, Ia.6, Ia.7 und Ia.8, worin E für Butan-1,4-diyl steht und m, R, R^{a} und R^{b} jeweils gemeinsam die in einer der Zeilen der Tabelle A angegebenen Bedeutungen aufweisen.

Unter den Verbindungen Ib sind die Verbindungen der nachfolgenden Formeln Ib.1, Ib.2, Ib.3 und Ib.4 besonders bevorzugt: 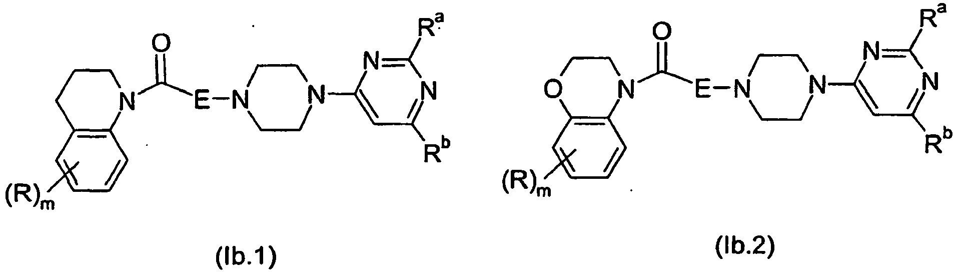 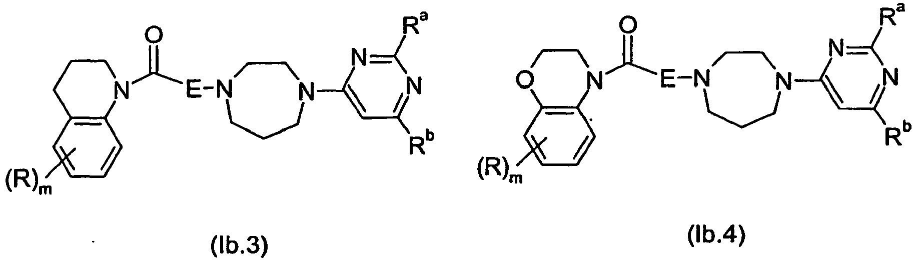 worin m, E, R, R^{a} und R^{b} die zuvor genannten Bedeutungen und insbesondere gemeinsam die als bevorzugt genannten Bedeutungen aufweisen.

Beispiele hierfür sind die Verbindungen der Formeln Ib.1, Ib.2, Ib.3 und Ib.4, worin E für Butan-1,4-diyl steht und m, R, R^{a} und R^{b} jeweils gemeinsam die in einer der Zeilen der Tabelle A angegebenen Bedeutungen aufweisen.

Unter den Verbindungen Ic sind die Verbindungen der nachfolgenden Formeln Ic.1 bis Ic.4 besonders bevorzugt: 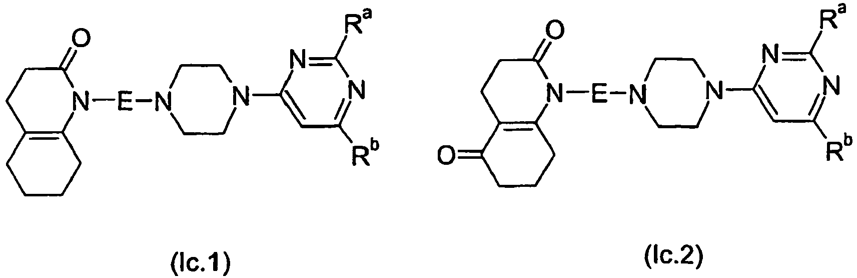 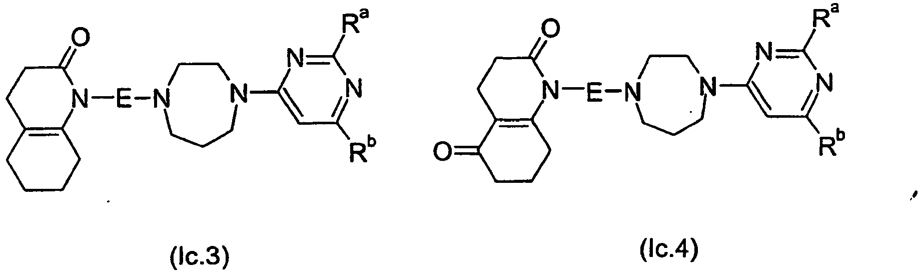 worin E, R^{a} und R^{b} die zuvor genannten Bedeutungen und insbesondere gemeinsam die als bevorzugt genannten Bedeutungen aufweisen.

Beispiele hierfür sind die Verbindungen der Formeln Ic.1, Ic.2, Ic.3 und Ic.4, worin E für Butan-1,4-diyl steht und R^{a} und R^{b} jeweils gemeinsam die in einer der Zeilen 1 bis 6 der Tabelle A angegebenen Bedeutungen aufweisen.

Unter den Verbindungen Id sind die Verbindungen der nachfolgenden Formeln Id.1 bis Id.6 besonders bevorzugt: 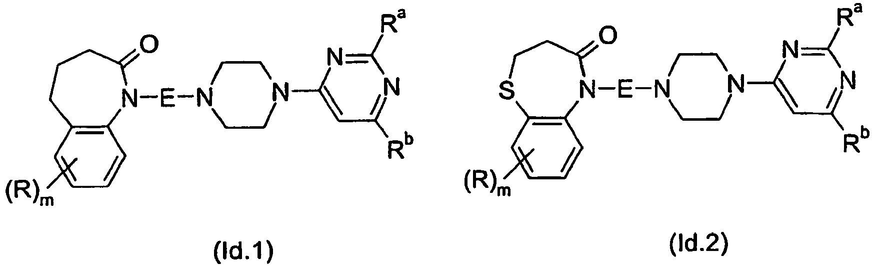 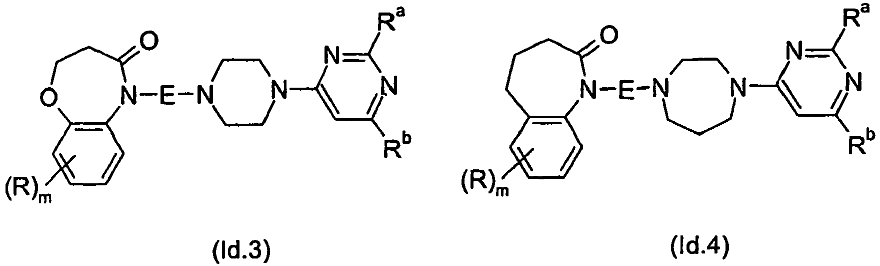 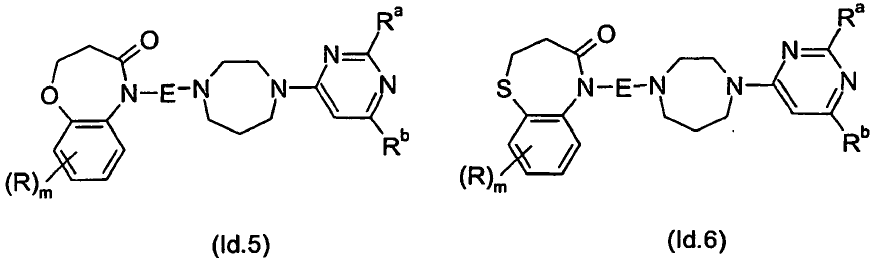 worin m, E, R, R^{a} und R^{b} die zuvor genannten Bedeutungen und insbesondere gemeinsam die als bevorzugt genannten Bedeutungen aufweisen.

Beispiele hierfür sind die Verbindungen der Formeln Id.1, Id.2, Id.3, Id.4, Id.5 und Id.6, worin E für Butan-1,4-diyl steht und m, R, R^{a} und R^{b} jeweils gemeinsam die in einer der Zeilen der Tabelle A angegebenen Bedeutungen aufweisen.

Unter den Verbindungen Ie sind die Verbindungen der nachfolgenden Formeln Ie.1 bis Ie.6 besonders bevorzugt: 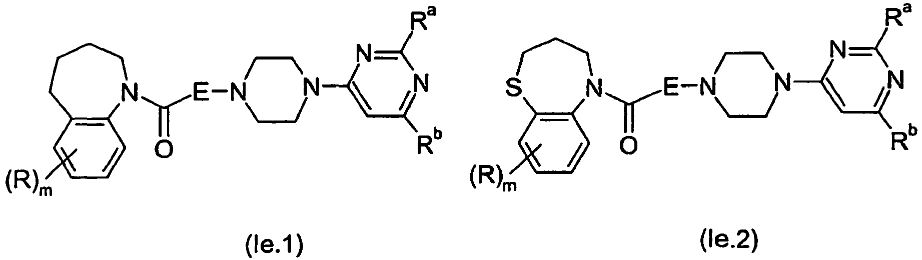 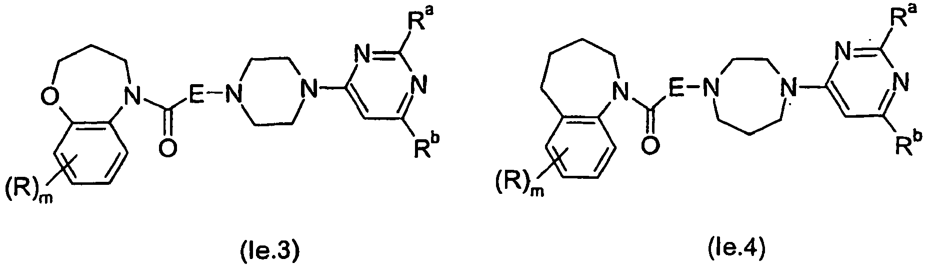 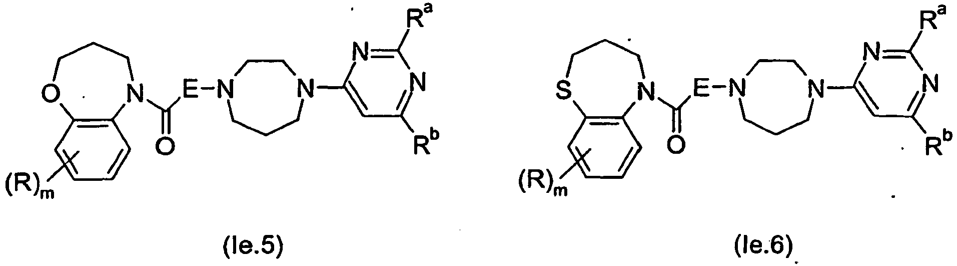 worin m, E, R, R^{e} und R^{b} die zuvor genannten Bedeutungen und insbesondere gemeinsam die als bevorzugt genannten Bedeutungen aufweisen.

Beispiele hierfür sind die Verbindungen der Formeln Ie.1, Ie.2, Ie.3, Ie.4, Ie.5 und Ie.6, worin E für Butan-1,4-diyl steht und m, R, R^{a} und R^{b} jeweils gemeinsam die in einer der Zeilen der Tabelle A angegebenen Bedeutungen aufweisen.

**Tabelle A:**

| Nr. | m | R | R^{a} | R^{b} |
|---|---|---|---|---|
| 1 | 0 | - | tert.-Butyl | CF₃ |
| 2 | 0 | - | tert.-Butyl | CHF₂ |
| 3 | 0 | - | tert.-Butyl | n-Propyl |
| 4 | 0 | - | tert.-Butyl | tert.-Butyl |
| 5 | 0 | - | tert.-Butyl | Cyclopropyl |
| 6 | 0 | - | tert.-Butyl | Cyclopentyl |
| 7 | 1 | α-Cl | tert.-Butyl | CF₃ |
| 8 | 1 | α-Cl | tert.-Butyl | CHF₂ |
| 9 | 1 | α-Cl | tert.-Butyl | n-Propyl |
| 10 | 1 | α-Cl | tert.-Butyl | tert.-Butyl |
| 11 | 1 | α-Cl | tert.-Butyl | Cyclopropyl |
| 12 | 1 | α-Cl | tert.-Butyl | Cyclopentyl |
| 13 | 1 | β-Cl | tert.-Butyl | CF₃ |
| 14 | 1 | β-Cl | tert.-Butyl | CHF₂ |
| 15 | 1 | β-Cl | tert.-Butyl | n-Propyl |
| 16 | 1 | β-Cl | tert.-Butyt | tert.-Butyl |
| 17 | 1 | β-Cl | tert.-Butyl | Cyclopropyl |
| 18 | 1 | β-Cl | tert.-Butyl | Cyclopentyl |
| 19 | 1 | γ-Cl | tert.-Butyl | CF₃ |
| 20 | 1 | γ-Cl | tert.-Butyl | CHF₂ |
| 21 | 1 | γ-Cl | tert.-Butyl | n-Propyl |
| 22 | 1 | γ-Cl | tert.-Butyl | tert.-Butyl |
| 23 | 1 | γ-Cl | tert.-Butyl | Cyclopropyl |
| 24 | 1 | γ-Cl | tert.-Butyl | Cyclopentyl |
| 25 | 1 | δ-Cl | tert.-Butyl | CF₃ |
| 26 | 1 | δ-Cl | tert.-Butyl | CHF₂ |
| 27 | 1 | δ-Cl | tert.-Butyl | n-Propyl |
| 28 | 1 | δ-Cl | tert.-Butyl | tert.-Butyl |
| 29 | 1 | δ-Cl | tert.-Butyl | Cyclopropyl |
| 30 | 1 | δ-Cl | tert.-Butyl | Cyclopentyl |
| 31 | 1 | α-OH | tert.-Butyl | CF₃ |
| 32 | 1 | α-OH | tert.-Butyl | CHF₂ |
| 33 | 1 | α-OH | tert.-Butyl | n-Propyl |
| 34 | 1 | α-OH | tert.-Butyl | tert.-Butyl |
| 35 | 1 | α-OH | tert.-Butyl | Cyclopropyl |
| 36 | 1 | α-OH | tert.-Butyl | Cyclopentyl |
| 37 | 1 | β-OH | tert.-Butyl | CF₃ |
| 38 | 1 | β-OH | tert.-Butyl | CHF₂ |
| 39 | 1 | β-OH | tert.-Butyl | n-Propyl |
| 40 | 1 | β-OH | tert.-Butyl | tert.-Butyl |
| 41 | 1 | β-OH | tert.-Butyl | Cyclopropyl |
| 42 | 1 | β-OH | tert.-Butyl | Cyclopentyl |
| 43 | 1 | γ-OH | tert.-Butyl | CF₃ |
| 44 | 1 | γ-OH | tert.-Butyl | CHF₂ |
| 45 | 1 | γ-OH | tert.-Butyl | n-Propyl |
| 46 | 1 | γ-OH | tert.-Butyl | tert.-Butyl |
| 47 | 1 | γ-OH | tert.-Butyl | Cyclopropyl |
| 48 | 1 | γ-OH | tert.-Butyl | Cyclopentyl |
| 49 | 1 | δ-OH | tert.-Butyl | CF₃ |
| 50 | 1 | δ-OH | tert.-Butyl | CHF₂ |
| 51 | 1 | δ-OH | tert.-Butyl | n-Propyl |
| 52 | 1 | δ-OH | tert.-Butyl | tert.-Butyl |
| 53 | 1 | δ-OH | tert.-Butyl | Cyclopropyl |
| 54 | 1 | δ-OH | tert.-Butyl | Cyclopentyl |
| 55 | 1 | α-OCH₃ | tert.-Butyl | CF₃ |
| 56 | 1 | α-OCH₃ | tert.-Butyl | CHF₂ |
| 57 | 1 | α-OCH₃ | tert.-Butyl | n-Propyl |
| 58 | 1 | α-OCH₃ | tert.-Butyl | tert.-Butyl |
| 59 | 1 | α-OCH₃ | tert.-Butyl | Cyclopropyl |
| 60 | 1 | α-OCH₃ | tert.-Butyl | Cyclopentyl |
| 61 | 1 | β-OCH₃ | tert.-Butyl | CF₃ |
| 62 | 1 | β-OCH₃ | tert.-Butyl | CHF₂ |
| 63 | 1 | β-OCH₃ | tert.-Butyl | n-Propyl |
| 64 | 1 | β-OCH₃ | tert.-Butyl | tert.-Butyl |
| 65 | 1 | β-OCH₃ | tert.-Butyl | Cyclopropyl |
| 66 | 1 | β-OCH₃ | tert.-Butyl | Cyclopentyl |
| 67 | 1 | γ-OCH₃ | tert.-Butyl | CF₃ |
| 68 | 1 | γ-OCH₃ | tert.-Butyl | CHF₂ |
| 69 | 1 | γ-OCH₃ | tert.-Butyl | n-Propyl |
| 70 | 1 | γ-OCH₃ | tert.-Butyl | tert.-Butyl |
| 71 | 1 | γ-OCH₃ | tert.-Butyl | Cyclopropyl |
| 72 | 1 | γ-OCH₃ | tert.-Butyl | Cyclopentyl |
| 73 | 1 | δ-OCH₃ | tert.-Butyl | CF₃ |
| 74 | 1 | δ-OCH₃ | tert.-Butyl | CHF₂ |
| 75 | 1 | δ-OCH₃ | tert.-Butyl | n-Propyl |
| 76 | 1 | δ-OCH₃ | tert.-Butyl | tert.-Butyl |
| 77 | 1 | δ-OCH₃ | tert.-Butyl | Cyclopropyl |
| 78 | 1 | δ-OCH₃ | tert.-Butyl | Cyclopentyl |
| 79 | 1 | α-CH₃ | tert.-Butyl | CF₃ |
| 80 | 1 | α-CH₃ | tert.-Butyl | CHF₂ |
| 81 | 1 | α-CH₃ | tert.-Butyl | n-Propyl |
| 82 | 1 | α-CH₃ | tert.-Butyl | tert.-Butyl |
| 83 | 1 | α-CH₃ | tert.-Butyl | Cyclopropyl |
| 84 | 1 | α-CH₃ | tert.-Butyl | Cyclopentyl |
| 85 | 1 | β-CH₃ | tert.-Butyl | CF₃ |
| 86 | 1 | β-CH₃ | tert.-Butyl | CHF₂ |
| 87 | 1 | β-CH₃ | tert.-Butyl | n-Propyl |
| 88 | 1 | β-CH₃ | tert.-Butyl | tert.-Butyl |
| 89 | 1 | β-CH₃ | tert.-Butyl | Cyclopropyl |
| 90 | 1 | β-CH₃ | tert.-Butyl | Cyclopentyl |
| 91 | 1 | γ-CH₃ | tert.-Butyl | CF₃ |
| 92 | 1 | γ-CH₃ | tert.-Butyl | CHF₂ |
| 93 | 1 | γ-CH₃ | tert.-Butyl | n-Propyl |
| 94 | 1 | γ-CH₃ | tert.-Butyl | tert.-Butyl |
| 95 | 1 | γ-CH₃ | tert.-Butyl | Cyclopropyl |
| 96 | 1 | γ-CH₃ | tert.-Butyl | Cyclopentyl |
| 97 | 1 | δ-CH₃ | tert.-Butyl | CF₃ |
| 98 | 1 | δ-CH₃ | tert.-Butyl | CHF₂ |
| 99 | 1 | δ-CH₃ | tert.-Butyl | n-Propyl |
| 100 | 1 | δ-CH₃ | tert.-Butyl | tert.-Butyl |
| 101 | 1 | δ-CH₃ | tert.-Butyl | Cyclopropyl |
| 102 | 1 | δ-CH₃ | tert.-Butyl | Cyclopentyl |
| 103 | 1 | β-C(O)CH₃ | tert.-Butyl | CF₃ |
| 104 | 1 | β-C(O)CH₃ | tert.-Butyl | CHF₂ |
| 105 | 1 | β-C(O)CH₃ | tert.-Butyl | n-Propyl |
| 106 | 1 | β-C(O)CH₃ | tert.-Butyl | tert.-Butyl |
| 107 | 1 | β-C(O)CH₃ | tert.-Butyl | Cyclopropyl |
| 108 | 1 | β-C(O)CH₃ | tert.-Butyl | Cyclopentyl |
| 109 | 1 | γ-C(O)CH₃ | tert.-Butyl | CF₃ |
| 110 | 1 | γ-C(O)CH₃ | tert.-Butyl | CHF₂ |
| 111 | 1 | γ-C(O)CH₃ | tert.-Butyl | n-Propyl |
| 112 | 1 | γ-C(O)CH₃ | tert.-Butyl | tert.-Butyl |
| 113 | 1 | γ-C(O)CH₃ | tert.-Butyl | Cyclopropyl |
| 114 | 1 | α-OC₂H₆ | tert.-Butyl | CF₃ |
| 115 | 1 | α-OC₂H₅ | tert.-Butyl | CHF₂ |
| 116 | 1 | α-OC₂H₅ | tert.-Butyl | n-propyl |
| 117 | 1 | α-OC₂H₅ | tert.-Butyl | tert.-Butyl |
| 118 | 1 | α-OC₂H₅ | tert.-Butyl | Cyclopropyl |
| 119 | 1 | α-OC₂H₅ | tert.-Butyl | Cyclopentyl |
| 120 | 1 | β-OC₂H₅ | tert.-Butyl | CF₃ |
| 121 | 1 | β-OC₂H₅ | tert.-Butyl | CHF₂ |
| 122 | 1 | β-OC₂H₅ | tert.-Butyl | n-Propyl |
| 123 | 1 | β-OC₂H₅ | tert.-Butyl | tert.-Butyl |
| 124 | 1 | β-OC₂H₅ | tert.-Butyl | Cyclopropyl |
| 125 | 1 | β-OC₂H₅ | tert.-Butyl | Cyclopentyl |
| 126 | 1 | γ-OC₂H₅ | tert.-Butyl | CF₃ |
| 127 | 1 | γ-OC₂H₅ | tert.-Butyl | CHF₂ |
| 128 | 1 | γ-OC₂H₅ | tert.-Butyl | n-Propyl |
| 129 | 1 | γ-OC₂H₅ | tert.-Butyl | tert.-Butyl |
| 130 | 1 | γ-OC₂H₅ | tert.-Butyl | Cyclopropyl |
| 131 | 1 | γ-OC₂H₅ | tert.-Butyl | Cyclopentyl |
| 132 | 1 | δ-OC₂H₅ | tert.-Butyl | CF₃ |
| 133 | 1 | δ-OC₂H₅ | tert.-Butyl | CHF₂ |
| 134 | 1 | δ-OC₂H₅ | tert.-Butyl | n-Propyl |
| 135 | 1 | δ-OC₂H₅ | tert.-Butyl | tert.-Butyl |
| 136 | 1 | δ-OC₂H₅ | tert.-Butyl | Cyclopropyl |
| 137 | 1 | δ-OC₂H₅ | tert.-Butyl | Cyclopentyl |

In Tabelle A geben die Bezeichungen α, β, γ, bzw. δ die Position des Substituenten R am Phenylring gemäß folgender Formel an.

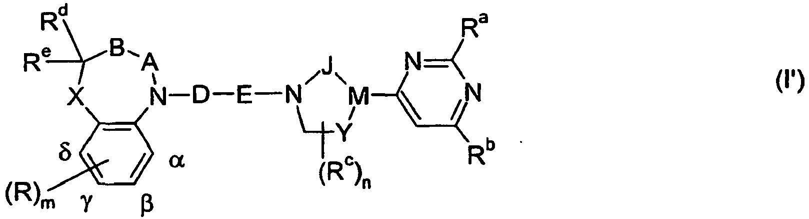

Die Herstellung der erfindungsgemäßen Verbindungen I kann in Analogie zu dem eingangs zitierten Stand der Technik erfolgen. Ein wichtiger Zugang zu den erfindungsgemäßen Verbindungen ist in Schema 1 dargestellt.

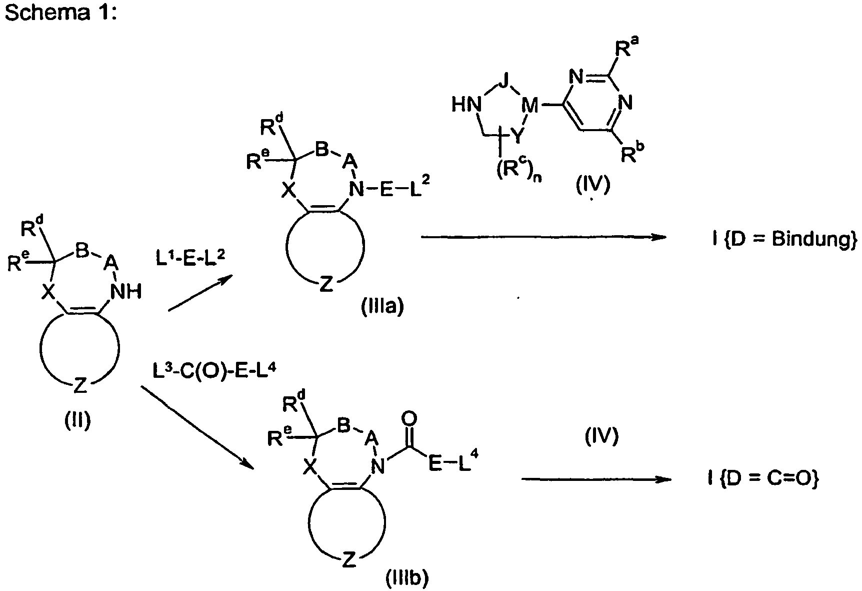

In Schema 1 haben n, A, B, D, E, X, Z, R^{a}, R^{b}, R^{c}, R^{d} und R^{e} die zuvor genannten Bedeutungen. L¹, L², L³ und L⁴ stehen für nucleophil verdrängbare Abgangsgruppen. Beispiele für geeignete nucleophil verdrängbare Abgangsgruppen L¹, L² und L⁴ sind Halogen, insbesondere Chlor, Brom oder Iod, Alkyl- und Arylsulfonat wie Mesylat, Tosylat. L³ steht beispielsweise für Halogen, insbesondere Chlor oder einen Aktivester-Rest, z.B. p-Nitrophenoxy. L¹ und L² sind vorzugsweise voneinander verschieden und weisen eine unterschiedliche Reaktivität auf. Beispielsweise steht L¹ für Brom oder Iod und L² für Chlor. Die für die Umsetzung erforderlichen Reaktionsbedingungen entsprechen den für nucleophile Substitutionen üblichen Reaktionsbedingungen.

Verbindungen der allgemeinen Formel IV sind entweder literaturbekannt, z.B. aus WO 96/02519, WO 97/25324, WO 99/02503, WO 00/42036, DE 10304870.7 oder der in diesen Schriften zitierten Literatur oder können nach den dort beschriebenen Verfahren hergestellt werden.

Die Verbindungen der Formel II sind ebenfalls bekannt und teilweise kommerziell erhältlich oder können in Analogie zu bekannten Verfahren hergestellt werden, wie beispielsweise beschrieben in: J. Org. Chem. 1981, 46(18), S. 3719, J. Heterocycl. Chem. 2001, 38(4), S. 961-964, J. Heterocycl. Chem. 1983, 20(3), S. 663.666, J. Med. Chem. 1986, 29(10), S. 1832-1840, J. Med. Chem. 2000 43, S. 3718-3735, J. Med. Chem. 1986, 29(1) S. 1-8, Chem. Pharm. Bull. 2000, 49(7), S.822-829, Org. Lett. 2002, 4(16), S. 2691-2694, DE 3800386 und EP-A 244697.

Die erfindungsgemäßen Verbindungen können z.T. auch nach den in den Schemata 2a bzw. 2b dargestellten Synthesen hergestellt werden:

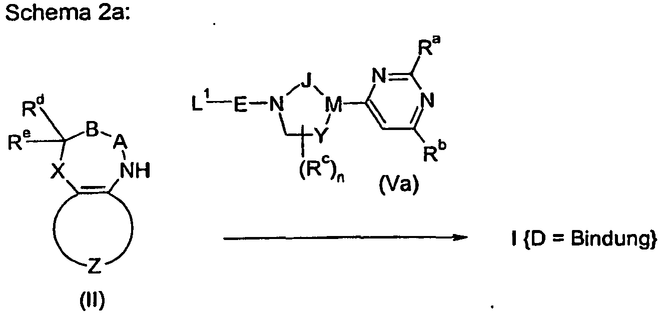

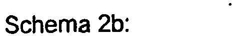

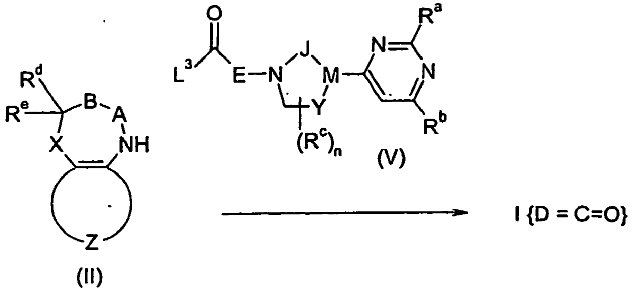

In den Schemata 2a bzw. 2b haben n, A, B, D, E, X, Z, R^{a}, R^{b}, R^{c}, R^{d} und R^{e} die zuvor genannten Bedeutungen. L¹ steht für eine nucleophil verdrängbare Abgangsgruppe. Beispielsweise steht L¹ für Chlor, Brom oder Iod. L³ steht üblicherweise für Halogen, insbesondere Chlor oder für den Rest einer Aktivestergruppe, z.B. für 4-Nitrophenoxy. Die für die Umsetzung erforderlichen Reaktionsbedingungen entsprechen den für nucleophile Substitutionen an Aliphaten bzw. die Umsetzung findet unter den für eine Amidierung von Säurehalogeniden üblichen Reaktionsbedingungen statt.

Verbindungen der allgemeinen Formel V sind ebenfalls literaturbekannt, z.B. aus WO 96/02519, WO 97/25324, WO 99/02503, WO 00/42036, DE 10304870.7 oder aus der in diesen Schriften zitierten Literatur oder können nach den dort beschriebenen Verfahren hergestellt werden, beispielsweise durch Umsetzung einer in Schema 1 gezeigten Verbindung der Formel IV mit einer Verbindung L³-C(O)-E-L⁴, worin L³, L⁴ und E die in Schema 1 angegebenen Bedeutungen aufweisen.

Verbindungen der Formel I, worin A für CH₂ steht, können außerdem durch Reduktion von Verbindungen I, worin A für C=O steht, hergestellt werden. Geeignete Reduktionsmittel umfassen beispielsweise Aluminiumhydride wie Lithiumaluminiumhydrid. Geeignete Methoden hierzu sind aus dem Stand der Technik bekannt, z.B. aus J. Org. Chem. 1972, 37, S. 2849 und können in analoger Weise für diese Umsetzung eingesetzt werden.

Sofern nichts anderes angegeben wird, erfolgen die oben beschriebenen Umsetzungen im allgemeinen in einem Lösungsmittel bei Temperaturen zwischen Raumtemperatur und der Siedetemperatur des verwendeten Lösungsmittels. Alternativ kann die zur Umsetzung erforderliche Aktivierungsenergie auch mittels Mikrowellen in die Reaktionsmischung eingetragen werden, was sich insbesondere bei den durch Übergangsmetalle katalysierten Reaktionen bewährt hat (zu Umsetzungen unter Einsatz von Mikrowellen siehe Tetrahedron 2001, 57, S. 9199 ff. S. 9225 ff. sowie allgemein "Microwaves in Organic Synthesis", André Loupy (Ed.), Wiley-VCH 2002).

Brauchbare Lösungsmittel sind beispielsweise Ether, wie Diethylether, Diisopropylether, Methyl-tert.-butylether oder Tetrahydrofuran, Halogenalkane wie Dichlormethan, Dichlorethan und dergleichen, Dimethylformamid, Dimethylsulfoxid, Dimethoxyethan, Toluol, Xylol, Acetonitril, Ketone, wie Aceton oder Methylethylketon, oder Alkohole, wie Methanol, Ethanol oder Butanol.

Gewünschtenfalls arbeitet man in Gegenwart einer Base zur Neutralisation der bei den Umsetzungen freiwerdenden Protonen. Geeignete Basen umfassen anorganische Basen, wie Natrium- oder Kaliumcarbonat, Natrium- oder Kaliumhydrogencarbonat, weiterhin Alkoholate wie Natriummethylat, Natriumethylat, Alkalimetallhydride wie Natriumhydrid, metallorganische Verbindungen, wie Butyllithium- oder Alkylmagnesium-Verbindungen, oder organische Stickstoffbasen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder durch Überführen in eine Säureadditionssalz.

Die Säureadditionssalze werden in üblicher Weise durch Mischen der freien Base mit der entsprechenden Säure, gegebenenfalls in Lösung in einem organischen Lösungsmittel, beispielsweise einem niedermolekularen Alkohol, wie Methanol, Ethanol oder Propanol, einem Ether, wie Methyl-tert-butylether, Diisopropylether, einem Keton, wie Aceton oder Methylethylketon oder einem Ester, wie Essigsäureethylester, hergestellt.

Bei den erfindungsgemäßen Verbindungen der Formel I handelt es sich in der Regel um hochselektive Dopamin-D₃-Rezeptor-Liganden, die auf Grund ihrer geringen Affinität gegenüber anderen Rezeptoren wie D₁-Rezeptoren, D₄-Rezeptoren, α1- und/oder α2-adrenergen-Rezeptoren, muskarinergen Rezeptoren, histaminischen Rezeptoren, Opiat-Rezeptoren und insbesondere gegenüber Dopamin-D₂-Rezeptoren, nebenwirkungsärmer als die klassischen Neuroleptika sind, bei denen es sich um D₂-Rezeptor-Antagonisten handelt.

Die hohe Affinität der erfindungsgemäßen Verbindungen gegenüber D₃-Rezeptoren spiegelt sich in sehr geringen in vitro Kᵢ-Werte von in der Regel weniger als 100 nM (nmol/l) häufig weniger als 50 nM und vor allem von weniger als 10 nM oder weniger als 5 nM wieder. Beispielsweise können Bindungsaffinitäten zu D₃-Rezeptoren in Rezeptorbindungsstudien über die Verdrängung von [¹²⁵I]-Iodosulpirid bestimmt werden.

Erfindungsgemäß von besonderer Bedeutung sind Verbindungen deren Selektivität Kᵢ(D₂)/Kᵢ(D₃) vorzugsweise wenigstens 10, besser noch wenigstens 30 und besonders verteilhaft wenigstens 50 beträgt. Rezeptorbindungsstudien an D₁-, D₂- und D₄-Rezeptoren können beispielsweise über die Verdrängung von [³H]SCH23390, [¹²⁵I]Iodosulpirid bzw. [¹²⁵I]Spiperon vorgenommen werden.

Die Verbindungen sind aufgrund ihres Bindungsprofils zur Behandlung von Erkrankungen brauchbar, die auf Dopamin-D₃-Liganden ansprechen, d. h. sie sind zur Behandlung von solchen Störungen bzw. Erkrankungen wirksam, bei denen eine Beeinflussung (Modulation) der Dopamin-D₃-Rezeptoren zur Verbesserung des Krankheitsbilds oder zum Ausheilen der Krankheit führt. Derartige Erkrankungen sind z. B. Störungen oder Erkrankungen des zentralen Nervensystems.

Unter Störungen bzw. Erkrankungen des zentralen Nervensystems versteht man Störungen, die das Rückenmark und vor allem das Gehirn betreffen. Der Begriff "Störung" im erfindungsgemäßen Sinne bezeichnet Anomalien, die in der Regel als krankhafte Zustände bzw. Funktionen angesehen werden und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefasst sein, die erfindungsgemäß behandelt werden können.

Zu den behandelbaren Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z. B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewusstseinsausfälle, -eintrübungen und -spaltungen und Persönlichkeitsstörungen; Störungen von Aufmerksamkeit und Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lem- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Eßstörungen, z.B. Anorexie oder Bulimie; Sucht; und weitere nicht näher bezeichnete psychiatrische Störungen.

Zu den behandelbaren Störungen gehören auch Parkinson und Epilepsie und insbesondere die damit in Zusammenhang stehenden affektiven Störungen.

Zu Suchterkrankungen gehören die durch den Missbrauch von psychotropen Substanzen, wie Arzneimittel oder Drogen, verursachte psychische Störungen und Verhaltensstörungen sowie andere Suchterkrankungen, wie beispielsweise die Spielsucht und die Impulsive-Kontrollstörung (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test.auch an Ratten beobachtet werden, die nach Verabreichung erfindungsgemäß brauchbarer Verbindungen die Selbstverabreichung von psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-D₃-Rezeptoren zurückzuführen sind.

Eine solche Behandlung richtet sich vor allem auf diejenigen Störungen, die sich über eine Bindung von vorzugsweise exogen zugesetzten Bindungspartnem (Liganden) an Dopamin-D₃-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit; Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontaneität und Entschlusskraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches.

Eine Behandlung umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des D₃-Rezeptors sind die erfindungsgemäßen Verbindungen I auch zur Behandlung von Nierenfunktionsstörungen, insbesondere von Nierenfunktionsstörungen, die durch Diabetes-Mellitus hervorgerufen wird (siehe WO 00/67847).

Die Verwendung der beschriebenen Verbindungen zur Behandlung beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabreichung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 1000 mg/kg Körpergewicht bei oraler Gabe bzw. von etwa 0,1 bis 100 mg/kg Körpergewicht bei parenteraler Gabe zugeführt wird.

Die Erfindung betrifft auch die Herstellung pharmazeutischer Mittel zur Behandlung eines Individuums, vorzugsweise eines Säugers, insbesondere eines Menschen, Nutz- oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Verbindungen verwendet werden. Ferner können auch Liposomen oder Mikrosphären zur Anwendung kommen.

Bei der Herstellung der Zusammensetzungen werden erfindungsgemäße Verbindungen gewöhnlich mit einem Exzipienten vermischt oder verdünnt. Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung.

Die magnetischen Kemresonanzspektraleigenschaften (NMR) beziehen sich auf chemische Verschiebungen (δ), ausgedrückt in Teile pro Million (ppm). Die relative Fläche für die Verschiebungen in dem ¹H-NMR-Spektrum entspricht der Anzahl der Wasserstoffatome für einen bestimmten funktionalen Typ in dem Molekül. Die Art der Verschiebung hinsichtlich der Multiplizität ist angegeben als Singulett (s), breites Singulett (s. br.), Dublett (d), breites Dublett (d br.), Triplett (t), breites Triplett (t br.), Quartett (q), Quintett (quint.), Multiplett (m). A) Herstellungsbeispiele:

### Beispiel 1

### 1-[2-tert-Butyl-6-(trifluormethyl)pyrimidin-4-yl]-4-[3-(2-oxo-3,4-dihydrochinolin-1(2H)-yl)propyl]piperazin-4-iumchlorid

Zu einer Suspension aus NaH (3,74 mmol, 0,15 g, 60%, entölt) in *N,N*-Dimethylformamid (10 ml) tropfte man bei 10 °C 3,4-Dihydrochinolin-2(1*H*)-on (3,40 mol, 0,50 g) in *N,N*-Dimethylformamid (5 ml) und aktivierte danach 1 Stunde bei Raumtemperatur vor. Anschließend tropfte man 2-*tert*-Butyl-4-[4-(3-chlorpropyl)piperazin-1-yl]-6-(trifluormethyl)pyrimidin (3,57 mmol, 1,30 g; hergestellt wie in DE 19728996) in N,N-Dimethylformamid (5 ml) zu. Das Reaktionsgemisch rührte man 12 Stunden bei Raumtemperatur nach. Nach Einengen des Reaktionsgemischs bis zur Trockne nahm man das verbliebene Öl in einem 1:1 Gemisch aus Ethylacetat/Wasser auf. Das wässrige Gemisch extrahierte man mit einer gesättigten Kochsalzlösung. Die organische Phase trocknete man über Na₂SO₄, filtrierte das Trockenmittel ab und engte die organische Phase unter vermindertem Druck ein. Den hierbei erhaltenen Rückstand reinigte man durch Chromatographie an Kieselgel (Eluierungsmittel: Heptan:Diethylether 0-100%), wobei man 840 mg der Titelverbindung erhielt.
ESI-MS: [M+Na⁺] = 498.2, [M+H⁺] = 477,2, 476,2, 238,6;
¹H-NMR (360 MHz, CDCl₃) δ (ppm): 13,41 (1H, s br.), 7,17 (1H, d), 7,12-6,97 (2H, m), 6,62 (1H, s), 4,53 (1H, s br.), 4,05 (2H, s br.), 3,95 (2H, s br.), 3,65 (2H, s br.), 3,13 (2H, s br.), 2,90 (2H, t), 2,81 (2H, m), 2,65 (2H, t), 2,40 (2H, s br.), 1,30 (9H, s).

### Beispiel 2

### 4-(3-{4-[2-tert-Butyl-6-(trifluormethyl)pyrimidin-4-yl]piperazin-1-yl}propyl)-2H-1,4-benzoxazin-3(4H)-on

Ausgehend von 2H-1,4-Benzoxazin-3(4*H*)-on (3,35 mmol, 0,50 g) erhielt man nach der in Beispiel 1 beschriebenen Methode 0,24 g der Titelverbindung.
ESI-MS: [M+Na⁺] = 500,1, 479,1, [M+H⁺] = 478,1, 239,6.
¹H-NMR (500 MHz, CDCl₃) δ (ppm): 7,09-6,97 (4H, m), 6,57 (1H, s), 4,60 (2H, s), 4,07 (2H, t), 3,69 (4H, s br.), 2,53-2,43 (4+2H, m), 1,88 (2H, quint.), 1,33 (9H, s). Beispiel 3

### 1-(4-{4-[2-tert-Butyl-6-(trifluormethyl)pyrimidin-4-yl]piperazin-1-yl}butyl)-3,4-dihydrochinolin-2(1H)-on

### 3.1 Gemisch aus 1-(4-Chlorbutyl)-3,4-dihydrochinolin-2(1H)-on und 1-(4-Brombutyl)-3,4-dihydrochinolin-2(1H)-on

Ausgehend von 3,4-Dihydrochinolin-2(1*H*)-on (6,79 mmol, 1,00 g) und 1-Brom-4-chlorbutan (8,15 mmol, 1,40 g) erhielt man nach der Beispiel 1 beschriebenen Methode 1,55 g des Gemischs aus 1-(4-Chlorbutyl)-3,4-dihydrochinolin-2(1*H*)-on und 1-(4-Brombutyl)-3,4-dihydrochinolin-2(1*H*)-on, das zu 10 % mit Edukt verunreinigt war.

### 3.2 1-(4-{4-[2-tert-Butyl-6-(trifluormethyl)pyrimidin-4-yl]piperazin-1-yl}butyl)-3,4-dihydrochinolin-2(1H)-on

Das Gemisch aus 1-(4-Chlorbutyl)-3,4-dihydrochinolin-2(1H)-on und 1-(4-Brombutyl)-3,4-dihydrochinolin-2(1H)-on (0,30 g), 2-tert-Butyl-4-piperazin-1-yl-6-(trifluormethyl)-pyrimidin (1,08 mmol, 0, 31 g; hergestellt wie in DE 19735410) und NEt3 (4,54 mmol, 0,46 g) in N,N-Dimethylformamid (10 ml) erwärmte man unter Rühren 20 Stunden auf 100 °C. Man ließ das Reaktionsgemisch abkühlen, versetzte mit Essigsäureethylester und wusch das Reaktionsgemisch zweimal mit Wasser. Die vereinten organischen Phasen trocknete man über Na₂SO₄, filtrierte das Trockenmittel ab und engte die organische Phase im Vakuum ein. Den erhaltenen öligen Rückstand reinigte man durch Chromatographie an Kieselgel (Eluierungsmittel: Methyl-tert.-butylether:Methanol 0-100%), wobei man 0,17 g der Titelverbindung erhielt.
ESI-MS: [M+Na⁺] = 512,5, 491,5, [M+H⁺] = 490,5;
¹H-NMR (500 MHz, CDCl₃) δ (ppm): 7,22 (1H, t+CHCl₃), 7,15 (1H, d), 7,05 (1H, d), 6,95 (1H, t), 6,58 (1H, s), 3,96 (2H, t), 3,70 (4H, s br.), 2,89 (2H, m), 2,65 (2H, m), 2,51 (4H, quint), 2,44 (2H, t), 1,71 (2H, quint), 1,61 (2H, quint.), 1,32 (9H, s).

### Beispiel 4

### 1-[2-tert-Butyl-6-(trifluormethyl)pyrimidin-4-yl]-4-[4-(3-oxo-2,3-dihydro-4H-1,4-benzoxazin-4-yl)butyl]piperazin-4-iumchlorid

### 4.1 Gemisch aus 4-(4-Chlorbutyl)-2H-1,4-benzoxazin-3(4H)-on und 4-(4-Brombutyl)-2H-1,4-benzoxazin-3(4H)-on

Ausgehend von 2*H*-1,4-Benzoxazin-3(4*H*)-on (33,52 mmol, 5,00 g) und 1-Brom-4-chlorbutan (40,23 mmol, 6,90 g) erhielt man nach der in Beispiel 1 beschriebenen Methode 7,50 g des Gemischs aus 4-(4-Chlorbutyl)-2*H*-1,4-benzoxazin-3(4*H*)-on und 4-(4-Brombutyl)-2*H*-1,4-benzoxazin-3(4*H*)-on.

### 4.2 1-[2-tert-Butyl-6-(trifluormethyl)pyrimidin-4-yl]-4-[4-(3-oxo-2,3-dihydro-4H-1,4-benzoxazin-4-yl)butyl]piperazin-4-iumchlorid

Ausgehend von dem Gemisch aus 4-(4-Chlorbutyl)-2*H*-1,4-benzoxazin-3(4*H*)-on und 4-(4-Brombutyl)-2*H*-1,4-benzoxazin-3(4*H*)-on (0,50 g) erhielt man nach der in Beispiel 3.2 beschriebenen Methode 0,96 g der Titelverbindung.
ESI-MS: 493,2, [M+H⁺] = 492,3, 246,6;
¹H-NMR (500 MHz, CDCl₃) δ (ppm): 7,05-6,96 (2+2H, m), 6,58 (1H, s), 4,59 (2H, s), 3,97 (2H, t), 3,71 (4H, s br.), 2,52 (4H, s br.), 2,45 (2H, m br.), 1,74 (2H, quint.), 1,64 (2H, quint.), 1,34 (9H, s).

### Beispiel 5

### 4-(4{4-[2-tert-Butyl-6-(trifluormethyl)pyrimidin-4-yl]perazin-1-yl}butyl)-6-methyl-2H-1,4-benzoxazin-3(4H)-on

### 5.1 Gemisch aus 4-(4-Chlorbutyl)-6-methyl-2H-1,4-benzoxazin-3(4H)-on und 4-(4-Brombutyl)-6-methyl-2H-1,4-benzoxazin-3(4H)-on

Ausgehend von 6-Methyl-2*H*-1,4-benzoxazin-3(4*H*)-on (15,69 mmol, 3,00 g; hergestellt wie in J. Heterocycl. Chem. 2001, 38(4), 961-4) und 1-Brom-4-chlorbutan (18,83 mmol, 3,23 g) erhielt man 4,72 g des Gemisches aus 4-(4-Chlorbutyl)-6-methyl-2*H*-1,4-benzoxazin-3(4*H*)-on und 4-(4-Brombutyl)-6-methyl-2*H*-1,4-benzoxazin-3(4*H*)-on.
ESI-MS:(Cl-Verbindung): [M+Na⁺] = 276,0, 256,0, [M+H⁺] = 254,16;
ESI-MS(Br-Verbindung): [M+K⁺] = 336,0, [M⁺H⁺] = 299,0, 298,0.

### 5.2 4-(4-{4-[2-tert-Butyl-6-(trifluormethyl)pyrimidin-4-yl]piperazin-1-yl}butyl)-6-methyl-2H-1,4-benzoxazin-3(4H)-on

Ausgehend von dem Gemisch aus 4-(4-Chlorbutyl)-6-methyl-2*H*-1,4-benzoxazin-3(4*H*)-on und 4-(4-Brombutyl)-6-methyl-2*H*-1,4-benzoxazin-3(4*H*)-on (0,50 g) erhielt man nach der in Beispiel 3.2 beschriebenen Methode 0,52 g der Titelverbindung.
ESI-MS: 507,2, [M+H⁺] = 506,2, 253,6;
¹H-NMR (500 MHz, CDCl₃) δ (ppm): 6,90-6,85 (1H, m), 6,80-6,75 (2H, m), 6,57 (1H, s), 4,54 (2H, s), 3,95 (2H, t), 3,69 (4H, s br.), 2,49 (4H, m sym.), 2,43 (2H, t), 2,34 (3H, s), 1,73 (2H, quint.), 1,65-1,58 (2H, m), 1,35 (9H, s).

### Beispiel 6

### 4-(4{4-[2-tert-Butyl-6-(trifluormethyl)pyrimidin-4-yl]piperazin-1-yl}butyl)-2H-1,4-benzothiazin-3(4H)-on

### 6.1 Gemisch aus 4-(4-Chlorbutyl)-2H-1,4-benzothiazin-3(4H)-on und 4-(4-Brombutyl)-2H-1,4-benzothiazin-3(4H)-on

Ausgehend von 2*H*-1,4-Benzothiazin-3(4*H*)-on (17,61 mmol, 3,00 g) und 1-Brom-4-chlorbutan (21,14 mmol, 3.62 g) erhielt man nach der in Beispiel 1 beschriebenen Methode 3,90 g des Gemisches aus 4-(4-Chlorbutyl)-2H-1,4-benzothiazin-3(4H)-on und 4-(4-Brombutyl)-2H-1,4-benzothiazin-3(4H)-on.
ESI-MS: (Cl-Verbindung): [M+Na⁺] = 278,0, 258,0, 257,0, [M+H⁺] = 256,0;
ESI-MS: (Br-Verbindung): [M+K⁺] = 340,0, [M+Na+] = 324,0, 302,0, [M+H⁺] = 301,0.

### 6.2 4-(4{4-[2-tert-Butyl-6-(trifluormethyl)pyrimidin-4-yl]piperazin-1-yl}butyl)-2H-1,4-benzothiazin-3(4H)-on

Ausgehend von dem Gemisch aus 4-(4-Chlorbutyl)-2H-1,4-benzothiazin-3(4H)-on und 4-(4-Brombutyl)-2H-1,4-benzothiazin-3(4H)-on (0,70 g)erhielt man nach der in Beispiel 3.2 beschriebenen Methode 0,31 g der Titelverbindung.
ESI-MS: 509,2, [M+H⁺] = 508,3, 254,6;
¹H-NMR (500 MHz, CDCl₃) δ (ppm): 7,35 (1H, d), 7,22 (1H + CHCl₃, t), 7,16 (1H, m), 7,00 (1H, t), 6,56 (1 H, s), 4,05 (2H, t), 3,68 (4H, s br.), 3,38 (2H, s), 2,46 (4H, m sym.), 2,39 (2H, t), 1,75 (2H, quint.), 1,65-1,52 (2H+H₂O, m), 1,33 (9H, s).

### Beispiel 7

### 4-(4-{4-[2-tert-Butyl-6-(trifluormethyl)pyrimidin-4-yl]piperazin-1-yl}butyl)-2-methyl-2H-1,4-benzoxazin-3(4H)-on

### 7.1 Gemisch aus 4-(4-Chlorbutyl)-2-methyl-2H-1,4-benzoxazin-3(4H)-on und 4-(4-Bromrbutyl)-2-methyl-2H-1,4-benzoxazin-3(4H)-on

Ausgehend von 2-Methyl-2*H*-1,4-benzoxazin-3(4*H*)-on (18,38 mmol, 3,00 g) und 1-Brom-4-chlorbutan (22,06 mmol, 3,78 g) erhielt man nach der in Beispiel 1 beschriebenen Methode 1,90 g des Gemischs aus 4-(4-Chlorbutyl)-2-methyl-2*H*-1,4-benzoxazin-3(4*H*)-on und 4-(4-Bromrbutyl)-2-methyl-2*H*-1,4-benzoxazin-3(4*H*)-on.
ESI-MS: (Cl-Verbindung): 256,1, 255,1, [M+H⁺] = 254,1;
ESI-MS: (Br-Verbindung): [M+K⁺] = 338,1, [M+Na⁺] = 322,1, 300,1, [M+H⁺] = 299,3, 254,1, 119,2.

### 7.2 4-(4-{4-[2-tert-Butyl-6-(trifluormethyl)pyrimidin-4-yl]piperazin-1-yl}butyl)-2-methyl-2H-1,4-benzoxazin-3(4H)-on

Ausgehend von dem Gemisch aus 4-(4-Chlorbutyl)-2-methyl-2H-1,4-benzoxazin-3(4H)-on und 4-(4-Brombutyl)-2-methyl-2H-1,4-benzoxazin-3(4H)-on (1,20 g) erhielt man nach der in Beispiel 3.2 beschriebenen Methode 1,00 g der Titelverbindung.
ESI-MS: 507,2, [M+H⁺] = 506,2, 253,6;
¹H-NMR (500 MHz, CDCl₃) ö (ppm): 7,03-6,96 (2+2H, m), 6,56 (1 H, s), 4,59 (1H, quart.), 3,96 (2H, t), 3,68 (4H, s br.), 2,48 (4H, m), 2,42 (2H, t), 1,73 (2H, m), 1,61 (2H, m), 1,56 (3H, d), 1,33 (9H, s).

### Beispiel 8

### 6-Acetyl-4-(4-{4-[2-tert-butyl-6-(trifluormethyl)pyrimidin-4-yl]piperazin-1-yl}butyl)-2H-1,4-benzoxazin-3(4H)-on

### 8.1 Gemisch aus 6-Acetyl-4-(4-chlorbutyl)-2H-1,4-benzoxazin-3(4H)-on und 6-Acetyl-4-(4-brombutyl)-2H-1,4-benzoxazin-3(4H)-on

Ausgehend von 6-Acetyl-2*H*-1,4-benzoxazin-3(4*H*)-on (15,69 mmol, 3,00 g) und 1-Brom-4-chlorbutan (18,83 mmol, 3,23 g) erhielt man nach der in Beispiel 1 beschriebenen Methode 3,40 g des Gemischs aus 6-Acetyl-4-(4-chlorbutyl)-2*H*-1,4-benzoxazin-3(4*H*)-on und 6-Acetyl-4-(4-brombutyl)-2*H*-1,4-benzoxazin-3(4*H*)-on.

### 8.2 6-Acetyl-4-(4-{4-[2-tert-butyl-6-(trifluormethyl)pyrimidin-4-yl]piperazin-1-yl}butyl)-2H-1,4-benzoxazin-3(4H)-on

Ausgehend von dem Gemisch aus 6-Acetyl-4-(4-chlorbutyl)-2*H*-1,4-benzoxazin-3(4*H*)-on und 6-Acetyl-4-(4-brombutyl)-2*H*-1,4-benzoxazin-3(4*H*)-on (1,40 g) erhielt man nach der in Beispiel 3.2 beschriebenen Methode 0,52 g der Titelverbindung.
ESI-MS: 535,2, [M+H⁺] = 534,2, 267,6;
¹H-NMR (500 MHz, CDCl₃) δ (ppm): 7,68 (1H, s), 7,60 (1H, d), 7,03 (1H, d), 6,57 (1H, s), 4,66 (2H, s), 4,05 (2H, t), 3,68 (4H, s br.), 2,59 (3H, s), 2,50 (4H, m sym.), 2,43 (2H, t), 1,75 (2H, quint.), 1,63 (2H, m), 1,33 (9H, s).

### Beispiel 9

### 1-(4-{4-[2-Cyclohexyl-6-(trifluormethyl)pyrimidin-4-yl]piperazin-1-yl}butyl)-3,4-dihydrochinolin-2(1H)-on

Ausgehend von dem Gemisch aus 1-(4-Chlorbutyl)-3,4-dihydrochinolin-2(1*H*)-on und 1-(4-Brombutyl)-3,4-dihydrochinolin-2(1*H*)-on (0,20 g) aus Beispiel 3.1 und 2-Cyclohexyl-4-piperazin-1-yl-6-(trifluormethyl)pyrimidin (0,88 mmol, 0,29 g, hergestellt wie in DE 19735410) erhielt man nach der in Beispiel 3.2 beschriebenen Methode 0,22 g der Titelverbindung.
ESI-MS: 517.3, [M+H⁺] = 516.3, 258.6.

### Beispiel 10

### 1-((2E)-4-{4-[2-tert-Butyl-6-(trifluormethyl)pyrimidin-4-yl]piperazin-1-yl}but-2-enyl)-3,4-dihydrochinolin-2(1 H)-on

Ausgehend von 3,4-Dihydrochinolinon (3,40 mmol, 0,50 g) und 2-tert-Butyl-4-{4-[(2E)-4-chlorbut-2-en-1-yl]piperazin-1-yl}-6-(trifluormethyl)pyrimidin (3,57 mmol, 1,34 g, hergestellt wie in DE 19728996) erhielt man nach der in Beispiel 1 beschriebenen Methode 1,35 g der Titelverbindung.
ESI-MS: 489,2, [M+H+] = 488,3, 244,6.

### Beispiel 11

### 1-(4-{4-[2-tert-Butyl-6-(trifluormethyl)pyrimidin-4-yl]piperazin-1-yl}butyl)-4,4-dimethyl-3,4-dihydrochinolin-2(1H)-on

### 11.1 Gemisch aus 1-(4-Chlorbutyl)-4,4-dimethyl-3,4-dihydrochinolin-2(1H)-on und 1-(4-Brombutyl)-4,4-dimethyl-3,4-dihydrochinolin-2(1H)-on (889-132)

Ausgehend von 4,4-Dimethyl-3,4-dihydrochinolin-2(1H)-on (11,41 mmol, 2,00 g; hergestellt wie in J. Med. Chem. 1986, 29, 1832-40) und 1-Brom-4-chlorbutan (13,70 mmol, 2,35 g) erhielt man 2,50 g des Gemischs aus 1-(4-Chlorbutyl)-4,4-dimethyl-3,4-dihydrochinolin-2(1*H*)-on und 1-(4-Brombutyl)-4,4-dimethyl-3,4-dihydrochinolin-2(1*H*)-on.
ESI-MS: (Cl-Verbindung): 269,1, 268,1, 266,1;
ESI-MS: (Br-Verbindung): 311,0, 310,0, 266,1.

### 11.2 1-(4-{4-[2-tert-Butyl-6-(trifluormethyl)pyrimidin-4-yl]piperazin-1-yl}butyl)-4,4-dimethyl-3,4-dihydrochinolin-2(1H)-on

Ausgehend von dem Gemisch aus 1-(4-Chlorbutyl)-4,4-dimethyl-3,4-dihydrochinolin-2(1 H)-on und 1-(4-Brombutyl)-4,4-dimethyl-3,4-dihydrochinolin-2(1H)-on (2,00 g) erhielt man 1,30 g der Titelverbindung.
ESI-MS: 519,3, [M+H⁺] = 518,35, 259,6.

### Beispiel 12

### 1-((2E)-4-{4-[2-tert-Butyl-6-(trifluormethyl)pyrimidin-4-yl]piperazin-1-yl}-2-methylbut-2-enyl)-3,4-dihydrochinolin-2(1H)-on

Ausgehend von 3,4-Dihydrochinolinon (3,40 mmol, 0,50 g) und 4-(2-tert-Butyl-6-trifluormethyl-pyrimidin-4-yl)-1-(4-chlor-3-methyl-but-2-enyl)-piperazin-1-iumchlorid (3,57 mmol, 1,52 g, hergestellt wie in DE 19728996) erhielt man nach der in Beispiel 1 beschriebenen Methode 0,05 g der Titelverbindung.
ESI-MS: 503,3, 502,2, [M+H+] = 251,6.

### Beispiel 13

### 6-Acetyl-1-(4-{4-[2-tert-butyl-6-(trifluormethyl)pyrimidin-4-yl]piperazin-1-yl}butyl)-4,4-dimethyl-3,4-dihydrochinolin-2(1H)-on

### 13.1 Gemisch aus 6-Acetyl-1-(4-chlorbutyl)-4,4-dimethyl-3,4-dihydrochinolin-2(1H)-on und 6-Acetyl-1-(4-brombutyl)-4,4-dimethyl-3,4-dihydrochinolin-2(1H)-on

Ausgehend von 6-Acetyl-4,4-dimethyl-3,4-dihydrochinolin-2(1H)-on (9,21 mmol, 2,00 g) und 1-Brom-4-chlorbutan (11,05 mmol, 1,89 g) erhielt man nach der in Beispiel 1 beschriebenen Methode 1,60 g des Gemisches aus 6-Acetyl-1-(4-chlorbutyl)-4,4-dimethyl-3,4-dihydrochinolin-2(1H)-on und 6-Acetyl-1-(4-brombutyl)-4,4-dimethyl-3,4-dihydrochinolin-2(1H)-on.

### 13.2 6-Acetyl-1-(4-{4-[2-tert-butyl-6-(trifluormethyl)pyrimidin-4-yl]piperazin-1-yl}butyl)-4,4-dimethyl-3,4-dihydrochinolin-2(1H)-on

Ausgehend von dem Gemisch aus 6-Acetyl-1-(4-chlorbutyl)-4,4-dimethyl-3,4-dihydrochinolin-2(1*H*)-on und 6-Acetyl-1-(4-brombutyl)-4,4-dimethyl-3,4-dihydrochinolin-2(1*H*)-on (3,25 mmol, 1,00 g) erhielt man nach der in Beispiel 3.2 beschriebenen Methode 0,01 g der Titelverbindung.
ESI-MS: 561,3, 560,3, [M+H¹] = 280,6.

### Beispiel 14

### 1-(4-{4-[2-tert-Butyl-6-(trifluormethyl)pyrimidin-4-yl]piperazin-1-yl}-2-methylbutyl)-3,4-dihydrochinolin-2(1H)-on

Zu einer Lösung von 1-((2*E*)4-{4-[2-*tert*-Butyl-6-(trifluormethyl)pyrimidin-4-yl]piperazin-1-yl}-2-methylbut-2-enyl)-3,4-dihydrochinolin-2(1*H*)-on (0,08 mmol, 49,99 mg) aus Beispiel 12 in Methanol (10 ml) gab man Pd/C (10%, 10,0 mg) und hydrierte anschließend 12 Stunden bei Raumtemperatur. Man erhielt 11,10 mg der Titelverbindung.
ESI-MS: 505,4, 504,4, [M+H⁺] = 252,7.

### Beispiel 15

### 1-(4-{4-[2-tert-Butyl-6-(trifluormethyl)pyrimidin-4-yl]piperazin-1-yl}pentyl)-3,4-dihydrochinolin-2(1H)-on

### 15.1 1-(4-Brompentyl)-3,4-dihydrochinolin-2(1H)-on

Ausgehend von 3,4-Dihydrochinolinon (8,83 mmol, 1,30 g) und 1,4-Dibrompentan (9,72 mmol, 2,30 g) erhielt man nach der in Beispiel 1 beschriebenen Methode 1,57 g der Titelverbindung.
¹H-NMR (500 MHz, CDCl₃) δ (ppm): 7,26 (1H, t), 7,17 (1H, d), 6,97 (2H, m), 4,17 (1H, m), 3,96 (2H, m), 2,87 (2H, m), 2,64 (2H, m), 1,95-1,74 (4H, m), 1,69 (3H, m).

### 15.2 1-(4-{4-[2-tert-Butyl-6-(trifluormethyl)pyrimidin-4-yl]piperazin-1-yl}pentyl)-3,4-dihydrochinolin-2(1H)-on

Ausgehend von 1-(4-Brompentyl)-3,4-dihydrochinolin-2(1H)-on (1,35 mmol, 0,40 g) erhielt man nach der in Beispiel 3.2 beschriebenen Methode 0,02 g der Titelverbindung.
ESI-MS: [M+Na⁺] = 526,2, 505,4, [M+H⁺] = 504,4, 252,6;
¹H-NMR (500 MHz, CDCl₃) δ (ppm): 7,21 (1H, t), 7,16 (1H, d), 7,07 (1 H, d), 6,98 (1H, t), 6,55 (1H, s), 4,04-3,96 (1 H, m), 3,93-3,84 (1 H, m), 3,65 (4H, s br.), 2,88 (2H, m), 2,73-2,58 (1+4H, m), 2,51-2,45 (2H, m), 1,72 (2H, quint.), 1,63-1,54 (1H, m), 1,45-1,37 (1H, m), 1,33 (9H, s), 0,95 (3H, d).

### Beispiel 16

### 1-(5-{4-[2-tert-Butyl-6-(trifluormethyl)pyrimidin-4-yl]piperazin-1-yl}pentyl)-3,4-dihydrochinolin-2(1H)-on

Ausgehend von 3,4-Dihydrochinolinon (0,82 mmol, 0,12 g) und 2-tert-Butyl-4-[4-(5-chlorpentyl)piperazin-1-yl]-6-(trifluormethyl)pyrimidin (0,77 mmol, 0,30 g; hergestellt wie in DE 19728996) erhielt man nach der in Beispiel 1 beschriebenen Methode 9,20 mg der Titelverbindung.
ESI-MS: 290,0, 274,1, 254,1, 253,1, 252,05.

### Beispiel 17

### 1-(4-{4-[2-tert-Butyl-6-(trifluormethyl)pyrimidin-4-yl]-1,4-diazepan-1-yl}butyl)-3,4-dihydrochinolin-2(1H)-on

Ausgehend von dem Gemisch aus 1-(4-Chlorbutyl)-3,4-dihydrochinolin-2(1 H)-on und 1-(4-Brombutyl)-3,4-dihydrochinolin-2(1H)-on (1,39 mmol, 0,33 g) aus Beispiel 3.1 und 1-(2-tert-Butyl-6-trifluormethyl-pyrimidin-4-yl)-[1,4]diazepan (1,32 mmol, 0,40 g, hergestellt wie in WO 97/25324) erhielt man nach der in Beispiel 3.2 beschriebenen Methode 0,17 g der Titelverbindung.
ESI-MS: 505,5, [M+H⁺] = 504,45, 252,7.

### Beispiel 18

### 4-[4-(2-tert-Butyl-6-trifluormethyl-pyrimidin-4-yl)-piperain-1-yl]-1-(3,4-dihydro-2H-chinolin-1-yl)-butan-1-on

### 18.1 4-Chlor-1-(3,4-dihydro-2H-chinolin-1-yl)-butan-1-on

Zu einer Suspension aus 1,2,3,4-Tetrahydrochinolin (30,03 mmol, 4,00 g), Kaliumcarbonat (44,15 mmol, 6,35 g) in Dioxan (100 ml) tropfte man bei 10 °C 5-Chlorbuttersäurechlorid (44,15 mmol, 6,35 g) in Dioxan (50 ml) zu. Man rührte die Reaktionsmischung 1 Stunde bei 10°C und anschließend 3 Stunden am Rückfluss nach. Nach dem Abfiltrieren der Salze engte man das Filtrat bis zur Trockne ein. Das verbliebene Öl nahm man in Dichlormethan auf und extrahierte das Gemisch dreimal mit einer 5%igen wässrigen NaHCO₃-Lösung (50 ml). Danach neutralisierte man mit 0,1 N HCl (20 ml) und wusch dreimal mit einer gesättigten Kochsalzlösung. Die organische Phase trocknete man über Na₂SO₄, filtrierte das Trockenmittel ab und engte bis zur Trockne ein, wobei man die Titelverbindung erhielt
ESI-MS: 240,1, [M+H⁺] = 238,1, 202,1.

### 18.2 4-[4-(2-tert-Butyl-6-trifluormethyl-pyrimidin-4-yl)-piperazin-1-yl]-1-(3,4-dihydro-2H-chinolin-1-yl)-butan-1-on

Ausgehend von 4-Chlor-1-(3,4-dihydro-2H-chinolin-1-yl)-butan-1-on (4,21 mmol, 1,00 g) erhielt man nach der in Beispiel 3.2 beschriebenen Methode 0,26 g der Titelverbindung.
ESI-MS: [M+H⁺] = 490,2, 245,6.

### Beispiel 19

### 1-(4-{4-[2-tert-Butyl-6-(trifluormethyl)pyrimidin-4-yl]piperazin-1-yl}butyl)-6-chlor-3,4-dihydrochinolin-2(1H)-on

### 19.1 6-Chlor-1-(4-chlorbutyl)-3,4-dihydrochinolin-2(1H)-on

Ausgehend von 6-Chlor-3,4-dihydrochinolin-2(1H)-on (11,01 mmol, 2,00 g; hergestellt wie in J. Med. Chem. 2000, 43, 3718-35) und 1-Brom-4-chlorbutan (13,21 mmol, 2,27 g) erhielt man nach der in Beispiel 1 beschriebenen Methode 2,90 g der Titelverbindung.

### 19.2 1-(4-{4-[2-tert-Butyl-6-(trifluormethyl)pyrimidin-4-yl]piperain-1-yl}butyl)-6-chloro-3,4-dihydrochinolin-2(1H)-on

Ausgehend von 6-Chlor-1-(4-chlorbutyl)-3,4-dihydrochinolin-2(1*H*)-on (4,96 mmol, 1,50 g) erhielt man nach der in Beispiel 3.2 beschriebenen Methode 1,34 g der Titelverbindung.
ESI-MS: 526,3, [M+H⁺] = 524,3, 263,8, 262,7.
¹H-NMR (500 MHz, CDCl₃) δ (ppm): 13,28 (1H, s br.), 7,22 (1H, d), 7,16 (1H, s), 6,90 (1H, d), 6,66 (1H, s), 4,54 (2H, s br.), 3,96 (2H, t), 3,63 (2H, s br.), 3,11 (2H, s br.), 2,91 (2H, t), 2,81 (2H, s br.), 2,63 (2H, t), 2.01 (2H, quint. br.), 1.76 (2H, quint.), 1.33 (9H, s).

### Beispiel 20

### 1-(4-{4-[2-tert-Butyl-6-(trifluormethyl)pyrimidin-4-yl]piperazin-1-yl}butyl)-6-methyl-3,4-dihydrochinolin-2(1H)-on

### 20.1 1-(4-Chlorbutyl)-6-methyl-3,4-dihydrochinolin-2(1H)-on

Ausgehend von 6-Methyl-3,4-dihydrochinolin-2(1H)-on (13.65 mmol, 2.20 g; hergestellt wie in J. Med. Chem. 2000, 43, 3718-35) und 1-Brom-4-chlorbutan (16.38 mmol, 2.81 g) erhielt man nach der in Beispiel 1 beschriebenen Methode 3.90 g der Titelverbindung.

### 20.2 1-(4-{4-[2-tert-Butyl-6-(trifluormethyl)pyrimidin-4-yl]piperazin-1-yl}butyl)-6-methyl-3,4-dihydrochinolin-2(1H)-on

Ausgehend von 1-(4-Chlorbutyl)-6-methyl-3,4-dihydrochinolin-2(1H)-on (6,75 mmol, 2,00 g) erhielt man nach der in Beispiel 3.2 beschriebenen Methode 0,56 g der Titelverbindung.
ESI-MS: 505,5, [M+H⁺] = 504,55, 252,9.

### Beispiel 21

### 1-(4-{4-[2-tert-Butyl-6-(trifluormethyl)pyrimidin-4-yl]piperazin-1-yl}butyl)-8-methoxy-3,4-dihydrochinolin-2(1H)-on

### 21.1 1-(4-Chlorbutyl)-8-methoxy-3,4-dihydrochinolin-2(1H)-n-on

Ausgehend von 8-Methoxy-3,4-dihydrochinolin-2(1*H*)-on (5,42 mmol, 1,20 g; hergestellt wie in Chem. Pharm. Bull. 2001, 49,822-9) und 1-Brom-4-chlorbutan (6,50 mmol, 1,12 g) erhielt man nach der in Beispiel 3.2 beschriebenen Methode 1,85 g der Titelverbindung.

### 21.2 1-(4-{4-[2-tert-Butyl-6-(trifluormethyl)pyrimidin-4-yl]piperazin-1-yl}butyl)-8-methoxy-3,4-dihydrochinolin-2(1H)-on

Ausgehend von 1-(4-Chlorbutyl)-8-methoxy-3,4-dihydrochinolin-2(1*H*)-on (5,68 mmol, 1,90 g) erhielt man nach der in Beispiel 3.2 beschriebenen Methode 1,25 g der Titelverbindung.
ESI-MS: [M+Na⁺] = 542,5, 521,5, [M+H⁺] = 520,5, 260,7.

### Beispiel 22

### 1-(4-{4-[2-tert-Butyl-6-(trifluormethyl)pyrimidin-4-yl]piperazin-1-yl}butyl)-8-hydroxy-3,4-dihydrochinolin-2(1H)-on

Die Demethylierung von 1-(4-{4-[2-*tert*-Butyl-6-(trifluormethyl)pyrimidin-4-yl]piperazin-1-yl}butyl)-8-methoxy-3,4-dihydrochinolin-2(1*H*)-on aus Beispiel 21 (1,35 mmol, 0,70 g) in Dichlormethan (40 ml) mit BBr₃ (1 M in Dichlormethan, 6,74 mmol, 1,67 g) über 12 Stunden bei Raumtemperatur ergab 0,41 g der Titelverbindung.
ESI-MS: 507,5, [M+H⁺] = 506,45, 253,8.

### Beispiel 23

### 5-[4-(2-tert-Butyl-6-trifluormethyl-pyrimidin-4-yl)-piperazin-1-yl]-1-(3,4-dihydro-2H-chinolin-1-yl)-pentan-1-on

### 23.1 5-Chlor-1-(3,4-dihydro-2H-chinolin-1-yl)-pentan-1-on

Ausgehend von 1,2,3,4-Tetrahydrochinolin (30,03 mmol, 4,00 g) und 5-Chlorvaleriansäurechlorid (45,05 mmol, 6,98 g) erhielt man nach der in Beispiel 18.1 beschriebene Methode 7,41 g der Titelverbindung.
ESI-MS: [M+H⁺] = 252,1.

### 23.2 5-[4-(2-tert-Butyl-6-trifluormethyl-pyrimidin-4-yl)-piperazin-1-yl]-1-(3,4-dihydro-2H-chinolin-1-yl)-pentan-1-on

Ausgehend von 5-Chlor1-(3,4-dihydro-2H-chinolin-1-yl)-pentan-1-on (7,94 mmol, 2,00 g) erhielt man nach der in Beispiel 3.2 beschriebenen Methode 0,45 g der Titelverbindung.
ESI-MS: [M+H⁺] = 504,5, 252,9.

### Beispiel 24

### 1-(4-{4-[2-tert-Butyl-6-(trifluormethyl)pyrimidin-4-yl]piperazin-1-yl}butyl)-8-methyl-3,4-dihydrochinolin-2(1H)-on

### 24.1 1-(4-Chlorbutyl)-8-methyl-3,4-dihydrochinolin-2(1H)-on

Ausgehend von 8-Methyl-3,4-dihydrochinolin-2(1H)-on (15,51 mmol, 2,50 g; hergestellt wie in J. Med. Chem. 2000, 43, 3718-35) und 1-Brom-4-chlorbutan (18,61 mmol, 3,19 g) erhielt man nach der in Beispiel 1 beschriebenen Methode 4,00 g der Titelverbindung.

### 24.2 1-(4-{4-[2-tert-Butyl-6-(trifluormethyl)pyrimidin-4-yl)piperazin-1-yl}butyl)-8-methyl-3,4-dihydrochinolin-2(1H)-on

Ausgehend von 1-(4-Chlorbutyl)-8-methyl-3,4-dihydrochinolin-2(1H)-on (5,96 mmol, 1,50 g) erhielt man nach der in Beispiel 3.2 beschriebenen Methode 1,00 g der Titelverbindung.
ESI-MS: [M+Na⁺] = 526,2, 505,4, [M+H⁺] = 504,4, 252,6;
¹H-NMR (500 MHz, DMSO-d₆) δ (ppm): 11,04 (1H, s br.), 7,18 (1H, s), 7,08 (2H, d), 6,97 (1 H, t), 3,86 (2H, t), 3,41 (4H, s), 2,96 (4H, m br.), 2,78 (2H, m sym), 2,40 (2H, m sym.), 2,27 (3H, s), 1,60 (2H, m sym.), 1,38 (2H, quint.), 1,28 (9H, s).

### Beispiel 25

### 1-(4-{4-[2-tert-Butyl-6-(trifluormethyl)pyrimidin-4-yl]piperazin-1-yl}butyl)-8-chlor-3,4-dihydrochinolin-2(1H)-on

### 25.1 8-Chlor-1-(4-chlorbutyl)-3,4-dihydrochinolin-2(1H)-on

Ausgehend von 8-Chlor-3,4-dihydrochinolin-2(1*H*)-on (27,53 mmol, 5,00 g; hergestellt wie in J. Med. Chem. 2000, 43, 3718-35) und 1-Brom-4-chlorbutan (33,04 mmol, 5,66 g) erhielt man nach der in Beispiel 1 beschriebenen Methode 8,05 g der Titelverbindung.

### 25.2 1-(4-{4-[2-tert-Butyl-6-(trifluormethyl)pyrimidin-4-yl]piperazin-1-yl}butyl)-8-chlor-3,4-dihydrochinolin-2(1H)-on

Ausgehend von 8-Chlor-1-(4-chlorbutyl)-3,4-dihydrochinolin-2(1H)-on (7,35 mmol, 2,00 g) erhielt man nach der in Beispiel 3.2 beschriebenen Methode 2,45 g der Titelverbindung.
ESI-MS: [M+H⁺] = 524,3, 262,6;
¹H-NMR (500 MHz, DMSO-d₆) δ (ppm): 11,21 (1 H, s br.), 7,33 (1 H, d), 7,25 (1 H, d), 7,18 (1 H, s), 7,08 (1 H, t), 4,06 (2H, m sym.), 3,46 (4H, s br.), 3,00 (4H, m), 2,88 (2H, m sym.), 2,53-2,47 (2H, m), 1,63 (2H, m sym.), 1,49 (2H, quint.), 1,30 (9H, s).

### Beispiel 26

### 1-(4-{4-[2-tert-Butyl-6-(trifluormethyl)pyrimidin-4-yl]piperazin-1-yl}butyl)-5-methyl-3,4-dihydrochinolin-2(1H)-on

### 26.1 1-(4-Chlorbutyl)-5-methyl-3,4-dihydrochinolin-2(1H)-on

Ausgehend von 5-Methyl-3,4-dihydrochinolin-2(1H)-on (6,20 mmol, 1,00 g; hergestellt wie in J. Med. Chem.2000, 43, 3718-35) und 1-Brom-4-chlorbutan (8,06 mmol, 1,38 g) erhielt man nach der in Beispiel 1 beschriebenen Methode 1,80 g der Titelverbindung.
ESI-MS: [M+H⁺] = 252,2.

### 26.2 1-(4-{4-[2-tert-Butyl-6-(trifluormethyl)pyrimidin-4-yl]piperazin-1-yl}butyl)-5-methyl-3,4-dihydrochinolin-2(1H)-on

Ausgehend von 1-(4-Chlorbutyl)-5-methyl-3,4-dihydrochinolin-2(1H)-on (3,57 mmol, 1,00 g) erhielt man nach der in Beispiel 3.2 beschriebenen Methode 0,50 g der Titelverbindung.
ESI-MS: [M+H⁺] = 504.4, 252.6.

### Beispiel 27

### 1-(4-{4-[2-tert-Butyl-6-(trifluormethyl)pyrimidin-4-yl]piperazin-1-yl}butyl)-6-methoxy-3,4-dihydrochinolin-2(1H)-on

### 27.1 6-Methoxy-3,4-dihydrochinolin-2(1H)-on

Bei Raumtemperatur behandelte man 6-Hydroxy-3,4-dihydrochinolin-2(1*H*)-on (18,38 mmol, 3,00 g) in Gegenwart von K₂CO₃ (22,98 mmol, 3,18 g) mit Methyliodid (22,98 mmol, 3,26 g) in N,N-Dimethylformamid (30 ml) 24 Stunden, wobei man 2,05 g der Titelverbindung erhielt.
ESI-MS: [M+H+] = 178,1.

### 27.2 Gemisch aus 1-(4-Chlorbutyl)-6-methoxy-3,4-dihydrochinolin-2(1H)-on und 1-(4-Brombutyl)-6-methoxy-3,4-dihydrochinolin-2(1H)-on

Ausgehend von 6-Methoxy-3,4-dihydrochinolin-2(1H)-on (14,11 mmol, 2,50 g) und 1-Brom-4-chlorbutan (21,16 mmol, 3,63 g) erhielt man nach der in Beispiel 1 beschriebenen Methode 3,12 g des Gemischs aus 1-(4-Chlorbutyl)-6-methoxy-3,4-dihydrochinolin-2(1 H)-on und 1-(4-Brombutyl)-6-methoxy-3,4-dihydrochinolin-2(1H)-on.
ESI-MS (Brom-Verbindung): 313,0, 312,0;
ESI-MS (Chlor-Verbindung): 270,1, 260,6.

### 27.3 1-(4-{4-[2-tert-Butyl-6-(trifluormethyl)pyrimidin-4-yl]plperazin-1-yl}butyl)-6-methoxy-3,4-dihydrochinolin-2(1H)-on

Ausgehend von dem Gemisch aus 1-(4-Chlorbutyl)-6-methoxy-3,4-dihydrochinolin-2(1*H*)-on und 1-(4-Brombutyl)-6-methoxy-3,4-dihydrochinolin-2(1*H*)-on (1,00 g) erhielt man nach der in Beispiel 3.2 beschriebenen Methode 0,70 g der Titelverbindung.
ESI-MS: [M+H⁺] = 520,3, 260,6;
¹H-NMR (500 MHz, CDCl₃) δ (ppm): 6,94 (1H, d), 6,78-6,70 (2H, m), 6,57 (1H, s), 3,93 (2H, t), 3,80 (3H, s), 3,70 (4H, s br.), 2,85 (2H, t), 2,62 (2H, t), 2,50 (4H, m sym.), 2,42 (2H, t), 1,72-1,63 (2H+ H₂O, m), 1,59 (2H, quint.), 1,35 (9H, s).

### Beispiel 28

### 1-(4-{4-[2-tert-Butyl-6-(trifluormethyl)pyrimidin-4-yl]piperazin-1-yl}butyl)-6-hydroxy-3,4-dihydrochinolin-2(1H)-on

Ausgehend von 1-(4-{4-[2-*tert*-Butyl-6-(trifluormethyl)pyrimidin-4-yl]piperazin-1-yl}butyl)-6-methoxy-3,4-dihydrochinolin-2(1*H*)-on aus Beispiel 27 (0,38 mmol, 0,20 g) erhielt man nach der in Beispiel 22 beschriebenen Methode 0,14 g der Titelverbindung.
¹H-NMR (500 MHz, DMSO-d₆) δ (ppm): 7,20 (1 H, s), 6,94 (1 H, d), 6,66-6,61 (2H, m), 3,85 (2H, m sym.), 3,09 (2H, s br.), 2,76 (2H, t), 2,52 (4H, s br.), 2,43 (2H, t), 1,71 (2H, m br.), 1,53 (2H, quint.), 1,31 (9H, s).

### Beispiel 29

### 1-(4-{4-[2-tert-Butyl-6-(trifluormethyl)pyrimidin-4-yl]piperazin-1-yl}butyl)-5-methoxy-3,4-dihydrochinolin-2(1H)-on

### 29.1 5-Methoxy-3,4-dihydrochinolin-2(1H)-on

Ausgehend von 5-Hydroxy-3,4-dihydrochinolin-2(1*H*)-on (12,26 mmol, 2,00 g) erhielt man nach der in Beispiel 27.1 beschriebenen Methode 1,80 g der Titelverbindung.
ESI-MS: [2M+H⁺] = 355,1, [M+H⁺] = 178,1;
¹H-NMR (500 MHz, DMSO-d₆) δ (ppm): 9,95 (1 H, s), 9,60 (1 H, s br.), 6,90 (1 H, t), 6,47 (1H, d), 6,35 (1 H, d), 2,76 (2H, t), 2,38 (2H, t).

### 29.2 Gemisch aus 1-(4-Chlorbutyl)-5-methoxy-3,4-dihydrochinolin-2(1H)-on und 1-(4-Brombutyl)-5-methoxy-3,4-dihydrochinolin-2(1H)-on

Ausgehend von 5-Methoxy-3,4-dihydrochinolin-2(1*H*)-on (9,11 mmol, 1,70 g) und 1-Brom-4-chlorbutan (13,67 mmol, 2,34 g) erhielt man nach der in Beispiel 1 beschriebenen Methode 2,80 g des Gemischs aus 1-(4-Chlorbutyl)-5-methoxy-3,4-dihydrochinolin-2(1*H*)-on und 1-(4-Brombutyl)-5-methoxy-3,4-dihydrochinolin-2(1*H*)-on.
ESI-MS: (Br-Verbindung): 334,1, 315,1, 312,1;
ESI-MS: (CI-Verbindung): 290,1, 270,2, 268,2.

### 29.3 1-(4-{4-[2-tert-Butyl-6-(trifluormethyl)pyrimidin-4-yl]piperazin-1-yl}butyl)-5-methoxy-3,4-dihydrochinolin-2(1H)-on

Ausgehend von dem Gemisch aus 1-(4-Chlorbutyl)-5-methoxy-3,4-dihydrochinolin-2(1*H*)-on und 1-(4-Brombutyl)-5-methoxy-3,4-dihydrochinolin-2(1*H*)-on (6,03 mmol, 1,70 g) erhielt man nach der in Beispiel 3.2 beschriebenen Methode 0,68 g der Titelverbindung.
ESI-MS: [M+Na⁺] = 542,5, 521,5, [M+H⁺] = 520,5, 260,9; .
¹H-NMR (500 MHz, DMSO-d₆) δ (ppm): 7,23-7,17 (1H, m), 6,78 (1H, d), 6,74 (1H, d), 3,91 (2H, m sym.), 3,78 (3H, s), 3,06 (4H, s br.), 2,77 (2H, t), 2,51-2,45 (4H, m), 1,71 (2H, m br.), 1,54 (2H, quint), 1,31 (9H, s).

### Beispiel 30

### 1-(4-{4-[2-tert-Butyl-6-(trifluormethyl)pyrimidin-4-yl]piperazin-1-yl}buty1)-7-methoxy-3,4-dihydrochinolin-2(1H)-on

### 30.1 1-(4-Chlorbutyl)-7-methoxy-3,4-dihydrochinolin-2(1H)-on

Ausgehend von 7-Methoxy-3,4-dihydrochinolin-2(1H)-on (11,74 mmol, 2,08 g; hergestellt wie in J. Med. Chem. 2000, 43, 3718-35) und 1-Brom-4-chlorbutan (14,09 mmol, 2,42 g) erhielt man nach der in Beispiel 1 beschriebenen Methode 3,93 g der Titelverbindung.
ESI-MS: [M+K⁺] = 306,1, [M+Na⁺] = 290,0, 270,1, [M+H⁺] = 268,1.

### 30.2 1-(4-{4-[2-tert-Butyl-6-(trifluormethyl)pyrimidin-4-yl]piperazin-1-yl}butyl)-7-methoxy-3,4-dihydrochinolin-2(1H)-on

Ausgehend von 1-(4-Chlorbutyl)-7-methoxy-3,4-dihydrochinolin-2(1H)-on (5,87 mmol, 1,97 g) erhielt man nach der in Beispiel 3.2 beschriebenen Methode 1,54 g der Titelverbindung.
ESI-MS: [M+Na⁺] = 542,5, [M+H⁺] = 521,5, 520,5, 260,7, 119,2;
¹H-NMR (400 MHz, CDCl₃) δ (ppm): 7,05 (1H, d), 6,66 (1H, s), 6,59 (1H, s), 6,52 (1H, d), 3,92 (2H, t), 3,76 (3H, s), 3,70 (4H, s br.), 2,81 (2H, t), 2,61 (2H, t), 2,50 (4H, t), 2,42 (2H, t), 1,70 (2H, m), 1,58 (2H, quint.), 1,33 (9H, s).

### Beispiel 31

### 1-(4-{4-[2-tert-Butyl-6-(trifluormethyl)pyrimidin-4-yl]piperazin-1-yl}butyl)-5-hydroxy-3,4-dihydrochinolin-2(1H)-on

Ausgehend von 1-(4-{4-[2-*tert*-Butyl-6-(trifluormethyl)pyrimidin-4-yl]piperazin-1-yl}butyl)-5-methoxy-3,4-dihydrochinolin-2(1*H*)-on aus Beispiel 29 (0,38 mmol, 0,20 g) erhielt man nach der in Beispiel 22 beschriebenen Methode 0,09 g der Titelverbindung.
ESI-MS: [M+Na⁺] = 528,3, 507,2, [M+H⁺] = 506,2, 253,6.

### Beispiel 32

### 1-(4-{4-[2-tert-Butyl-6-(trifluormethyl)pyrimidin-4-yl]piperazin-1-yl}butyl)-7-hydroxy-3,4-dihydrochinolin-2(1H)-on

Ausgehend von 1-(4-{4-[2-*tert*-Butyl-6-(trifluormethyl)pyrimidin-4-yl]piperazin-1-yl}butyl)-7-methoxy-3,4-dihydrochinolin-2(1*H*)-on aus Beispiel 30 (0,94 mmol, 0,49 g) erhielt man nach der in Beispiel 22 beschriebenen Methode 0,39 g der Titelverbindung.
ESI-MS: [M+H⁺] = 506,2, 253,6;
¹H-NMR (400 MHz, CDCl₃) δ (ppm): 6,98 (1 H, d), 6,62 (1H, s), 6,58 (1H, s), 6,44 (1 H, d), 3,90 (2H, t), 3,71 (4H, s br.), 2,78 (2H, t), 2,60 (2H, t), 2,52 (4H, m), 2,43 (2H, t), 1,71 (2H, quint.), 1,60 (2H, quint.), 1,33 (9H, s).

### Beispiel 33

### 1-{4-[4-(2-tert-Butyl-6-propylpyrimidin-4-yl)piperazin-1-yl]butyl}-5-methoxy-3,4-dihydrochinolin-2(1H)-on

Ausgehend von dem Gemisch aus 1-(4-Chlorbutyl)-5-methoxy-3,4-dihydrochinolin-2(1*H*)-on und 1-(4-Brombutyl)-5-methoxy-3,4-dihydrochinolin-2(1*H*)-on aus Beispiel 29.2 (0,50 g) und 2-*tert*-Butyl-4-piperain-1-yl-6-propylpyrimidin (1,77 mmol, 0,47 g; hergestellt wie in DE 19735410) erhielt man nach der in Beispiel 3.2 beschriebenen Methode 0,40 g der Titelverbindung.
ESI-MS: [M+H⁺] = 494,5, 247,9;
¹H-NMR (400 MHz, CDCl₃) δ (ppm): 7,17 (1H, t), 6,70 (1H, d), 6,61 (1H, d), 6,11 (1H, s), 3,93 (2H, t), 3,83 (3H, s), 3,61 (4H, s br.), 2,88 (2H, t), 2,57 (2H, t), 2,53 (2H, t), 2,47 (4H, m), 2,39 (2H, t), 1,76-1,53 (9H, m), 1,31 (9H, s), 0,94 (3H, t).

### Beispiel 34

### 1-{4-[4-(2-tert-Butyl-6-propylpyrimidin-4-yl)piperazin-1-yl]butyl}-5-hydroxy-3,4-dihydrochinolin-2(1H)-on

Ausgehend von 1-{4-[4-(2-tert-Butyl-6-propylpyrimidin-4-yl)piperazin-1-yl]butyl}-5-methoxy-3,4-dihydrochinolin-2(1H)-on aus Beispiel 33 (0,59 mmol, 0,29 g) erhielt man nach der in Beispiel 22 beschriebenen Methode 0,03 g der Titelverbindung.
ESI-MS: 481,5, 480,5, 240,9;
¹H-NMR (400 MHz, CDCl₃) δ (ppm): 7,02 (1 H, t), 6,61 (1H, d), 6,50 (1 H, d), 6,13 (1 H, s), 3,94 (2H, t), 3,64 (4H, s br.), 2,88 (2H, t), 2,58 (2H, t), 2,56-2,47 (4H, m), 2,42 (2H, t), 1,84-1,52 (6H, m), 1,32 (9H, s), 1,26 (2H, t), 0,93 (4H, t).

### Beispiel 35

### 1-(4-{4-[2-tert-Butyl-6-(difluormethyl)pyrimidin-4-yl]piperazin-1-yl}butyl)-5-methoxy-3,4-dihydrochinolin-2(1H)-on

Ausgehend von dem Gemisch aus 1-(4-Chlorbutyl)-5-methoxy-3,4-dihydrochinolin-2(1 H)-on und 1-(4-Brombutyl)-5-methoxy-3,4-dihydrochinolin-2(1 H)-on aus Beispiel 29.2 (0,50 g) und 2-tert-Butyl-4-piperazin-1-y1-6-difluormethylpyrimidin (1,87 mmol, 0,51 g; hergestellt wie in DE 19735410) erhielt man nach der in Beispiel 3.2 beschriebenen Methode 0,16 g der Titelverbindung.
ESI-MS: [M+H⁺] = 502.2, 251.8;
¹H-NMR (400 MHz, CDCl₃) δ (ppm): 7,19 (1H, t), 6,71 (1H, d), 6,63 (1H, d), 6,55 (1 H, s), 6,36 (1 H, s), 3,95 (2H, t), 3,84 (3H, s), 3,68 (4H, s br.), 2,88 (2H, t), 2,58 (2H, t), 2,49 (4H, m), 2,42 (2H, t), 1,69 (2H, quint.), 1,57 (2H, quint.), 1,33 (9H, s).

### Beispiel 36

### 1-(4-{4-[2-tert-Butyl-6-(difluormethyl)pyrimidin-4-yl]piperazin-1-yl}butyl)-5-hydroxy-3,4-dihydrochinolin-2(1H)-on

Ausgehend von 1-(4-{4-[2-*tert*-Butyl-6-(difluormethyl)pyrimidin-4-yl]piperazin-1-yl}butyl)-5-methoxy-3,4-dihydrochinolin-2(1*H*)-on aus Beispiel 35 (0,24 mmol, 0,12 g) erhielt man nach der in Beispiel 22 beschriebenen Methode 0,03 g der Titelverbindung.
ESI-MS: [M+H⁺] = 488,4, 244,8.

### Beispiel 37

### 1-{4-[4-(2-tert-Butyl-6-propylpyrimidin-4-yl)piperazin-1-yl]butyl}-3,4-dihydrochinolin-2(1H)-on

Ausgehend von 1-(4-Chlorbutyl)-3,4-dihydrochinolin-2(1H)-on und 1-(4-Brombutyl)-3,4-dihydrochinolin-2(1H)-on aus Beispiel 3.1 (0,59 g) und 2-tert-Butyl-4-piperazin-1-yl-6-propylpyrimidin (2,38 mmol, 0,62 g, hergestellt wie in DE 19735410) erhielt man nach der in Beispiel 3.2 beschriebenen Methode 0,82 g der Titelverbindung.
ESI-MS: [M+H⁺] = 464,4, 232,7;
¹H-NMR (400 MHz, CDCl₃) δ (ppm): 7,22 (1 H, t), 7,16 (1 H, d), 7,05 (1H, d), 7,00 (1 H, t), 6,13 (1H, s), 3,96 (2H, t), 3,61 (4H, s br.), 2,88 (2H, t), 2,64 (2H, t), 2,59-2,28 (7H, m), 1,76-1,49 (8H, m), 1,33 (9H, s), 0,94 (2H, t).

### Beispiel 38

### 1-{4-[4-(2-tert-Butyl-6-propylpyrimidin-4-yl)piperazin-1-yl]butyl}-8-methoxy-3,4-dihydrochinolin-2(1H)-on

Ausgehend von 1-(4-Chlorbutyl)-8-methoxy-3,4-dihydrochinolin-2(1*H*)-on aus Beispiel 21.1 (3,36 mmol, 1,00 g) und 2-*tert*-Butyl-4-piperazin-1-yl-6-propylpyrimidin (3,36 mmol, 0,88 g; hergestellt wie in DE 19735410) erhielt man nach der in Beispiel 3.2 beschriebenen Methode 0,43 g der Titelverbindung.
ESI-MS: 495.4, 494.4, [M+H⁺] = 321,2, 247,7;
¹H-NMR (400 MHz, CDCl₃) δ (ppm): 7,00 (1H, t), 6,80 (1H, d), 6,77 (1H, d), 6,10 (1H,
s), 4,07 (2H, t), 3,85 (3H, s), 3,59 (4H, s br.), 2,80 (2H, m), 2,64-2,23 (9H, m incl. 2,32 (2H, t)), 1,69 (2H, sext.), 1,63-1,50 (6H, m), 1,45 (2H, m), 1,31 (9H, s), 0,95 (3H, t).

### Beispiel 39

### 1-{4-[4-(2-tert-Butyl-6-propylpyrimidin-4-yl)piperazin-1-yl]butyl}-8-hydroxy-3,4-dihydrochinolin-2(1H)-on

Ausgehend von 1-{4-[4-(2-*tert*-Butyl-6-propylpyrimidin-4-yl)piperazin-1-yl]butyl}-8-methoxy-3,4-dihydrochinolin-2(1*H*)-on aus Beispiel 38 (0,20 mmol, 0,10 g) erhielt man nach der in Beispiel 22 beschriebenen Methode 42,00 mg der Titelverbindung.
ESI-MS: 480,4, [M+H⁺] = 240,7.

### Beispiel 40

### 1-{4-[4-(2-tert-Butyl-6-propylpyrimidin-4-yl)piperazin-1-yl]butyl}-5-ethoxy-3,4-dihydrochinolin-2(1H)-on

### 40.1 5-Ethoxy-3,4-dihydrochinolin-2(1H)-on

Ausgehend von 5-Hydroxy-3,4-dihydrochinolin-2(1*H*)-on (15,32 mmol, 2,50 g) und Ethyliodid (30,64 mmol, 4,78 g) erhielt man nach der in Beispiel 27.1 beschriebenen Methode 2,80 g der Titelverbindung.
ESI-MS: [M+H⁺] = 192,1.

### 40.2 Gemisch aus 1-(4-Chlorbutyl)-5-ethoxy-3,4-dihydrochinolin-2(1H)-on und 1-(4-Brombutyl)-5-ethoxy-3,4-dihydrochinolin-2(1H)-on

Ausgehend von 5-Ethoxy-3,4-dihydrochinolin-2(1*H*)-on (2,61 mmol, 0,50 g) und 1-Brom-4-chlorbutan (3,14 mmol, 0,54 g) erhielt man nach der in Beispiel 1 beschriebenen Methode 0,67 g der Titelverbindung.
ESI-MS:(Br-Verbindung): 284,1, 282,1;
ESI-MS:(CI-Verbindung): 282,1, 280,1, 246,1, 102,2.

### 40.3 1-{4-[4-(2-tert-Butyl-6-propylpyrimidin-4-yl)piperazin-1-yl]butyl}-5-ethoxy-3,4-dihydrochinolin-2(1H)-on

Ausgehend von dem Gemisch aus 1-(4-Chlorbutyl)-5-ethoxy-3,4-dihydrochinolin-2(1*H*)-on und 1-(4-Brombutyl)-5-ethoxy-3,4-dihydrochinolin-2(1*H*)-on (0,10 g) und 2-*tert*-Butyl-4-piperazin-1-yl-6-propylpyrimidin (0,35 mmol, 93.13m g; hergestellt wie in DE19735410) erhielt man nach der in Beispiel 3.2 beschriebenen Methode 0,16 g der Titelverbindung.
ESI-MS: 508,4, [M+H⁺] = 254,8;
¹H-NMR (400 MHz, CDCl₃) δ (ppm): 7,15 (1H, t), 6,69 (1H, d), 6,60 (1H, d), 6,14 (1H, s), 4,04 (2H, q), 3,94 (2H, t), 3,63 (4H, s br.), 2,88 (2H, t), 2,68-2,26 (10H, m), 1,71 (2H, quint.), 1,59 (4H, s br.), 1,44 (3H, t), 1,32 (9H, s), 0,96 (3H, t).

### Beispiel 41

### 1-{4-[4-(2-tert-Butyl-6-trifluormethyl-pyrimidin-4-yl)-piperazin-1-yl]-butyl}-5-ethoxy-3,4-dihydro-1H-chinolin-2-on

Ausgehend von dem Gemisch aus 1-(4-Chlorbutyl)-5-ethoxy-3,4-dihydrochinolin-2(1*H*)-on und 1-(4-Brombutyl)-5-ethoxy-3,4-dihydrochinolin-2(1*H*)-on aus Beispiel 40.2 (0,05 g) erhielt man nach der in Beispiel 3.2 beschriebenen Methode 46,00 mg der Titelverbindung.
ESI-MS: [M+Na⁺] = 556,3, 535,3, [M+H⁺] = 534,3, 267,6;
¹H-NMR (400 MHz, DMSO-d₆) δ (ppm): 7,19 (1 H, t), 7,03 (1 H, s), 6,79 (1H, d),6,69 (1 H, d), 4,03 (2H, quart.), 3,87 (2H, t), 3,68 (3H, s br.), 2,77 (2H, t), 2,63-2,20 (6H, m), 1,53 (4H, m sym.), 1,32 (3H, t), 1,27 (9H, s).

### Beispiel 42

### 1-{4-[4-(2-tert-Butyl-6-trifluormethyl-pyrimidin-4-yl)-piperazin-1-yl]-butyl}-3,4,5,6,7,8-hexahydro-1H-chinolin-2-on

Ausgehend von 1-(4-Chlorbutyl)-3,4,5,6,7,8-hexahydro-1H-chinolin-2-on (hergestellt durch Umsetzung von 3,4,5,6,7,8-Hexahydro-1H-chinolin-2-on mit 1-Brom-4-chlorbutan nach der Vorschrift in Beispiel 1) erhielt man nach der in Beispiel 3.2 beschriebenen Methode die Titelverbindung.
ESI-MS: [M+Na⁺] = 516,3, 495,4, [M+H⁺] = 494,4, 247,6;
¹H-NMR (500 MHz, CDCl₃) δ (ppm): 6,56 (1H, s), 3,69 (4H, s br.), 3,57 (2H, m sym.), 2,53-2,37 (8H, m), 2,14 (2H, m sym.), 2,11-2,02 (2H, m), 1,74-1,62 (4H, m), 1,62-1,46 (6H, m), 1,34 (9H, s).

### Beispiel 43

### 1-{4-[4-(2-tert-Butyl-6-trifluormethyl-pyrimidin-4-yl)-piperazin-1-yl]-butyl}-1H-chinolin-2-on

### 43.1 Gemisch aus 1-(4-Chlorbutyl)-1H-chinolin-2-on und 1-(4-Brombutyl)-1H-chinolin-2-on

Nach der in Beispiel 3.1 beschriebenen Methode erhielt man das Gemisch aus 1-(4-Chlorbutyl)-1 H-chinolin-2-on und 1-(4-Brombutyl)-1H-chinolin-2-on.
ESI-MS (CI-Verbindung): 238,1, 236,1;
ESI-MS (Br-Verbindung): [M+Na⁺] = 302,0, [M+H⁺] = 280,0,236,1.

### 43.2 1-{4-[4-(2-tert-Butyl-6-trifluormethyl-pyrimidin-4-yl)-piperazin-1-yl]-butyl}-1H-chinolin-2-on

Ausgehend von dem Gemisch aus 1-(4-Chlor-butyl)-1H-chinolin-2-on und 1-(4-Brom-butyl)-1 H-chinolin-2-on erhielt man nach der in Beispiel 3.2 beschriebenen Methode die Titelverbindung.
ESI-MS: [M+Na⁺] = 510,2, 489,2, [M+H⁺] = 488,3, 244,6;
¹H-NMR (500 MHz, CDCl₃) δ (ppm): 7,66 (1H, d), 7,58-7,51 (2H, m), 7,43 (1H, d), 7,20 (1 H, t), 6,69 (1 H, d), 6,56 (1H, s), 4,33 (2H, t), 3,68 (4H, s br.), 2,51 (4H, m sym.), 2,45 (2H, t), 1,82 (2H, quint.), 1,68 (2H, quint.), 1,34 (9H, s).

### Beispiel 44

### 1-{4-[4-(2-tert-Butyl-6-propyl-pyrimidin-4-yl)-piperazin-1-yl]-butyl}-4,6,7,8-tetrahydro-1H,3H-chinoline-2,5-dion

### 44.1 Gemisch aus 1-(4-Chlorbutyl)-4,6,7,8-tetrahydro-1H,3H-chinolin-2,5-dion und 1-(4-Brombutyl)-4,6,7,8-tetrahydro-1H,3H-chinoline-2,5-dion

Nach der in Beispiel 3.1 beschriebenen Methode erhielt man das Gemisch aus 1-(4-Chlorbutyl)-4,6,7,8-tetrahydro-1H,3H-chinolin-2,5-dion und 1-(4-Brombutyl)-4,6,7,8-tetrahydro-1H,3H-chinoline-2,5-dion.
ESI-MS (Cl-Verbindung): 258,1, 256,1;
ESI-MS (Br-Verbindung): 301,0, 300,0.

### 44.2 1-{4-[4-(2-tert-Butyl-6-propyl-pyrimidin-4-yl)-piperazin-1-yl]-butyl}-4,6,7,8-tetrahydro-1H,3H-chinoline-2,5-dion

Ausgehend von dem Gemisch 1-(4-Chlorbutyl)-4,6,7,8-tetrahydro-1 H,3H-chinolin-2,5-dion und 1-(4-Brombutyl)-4,6,7,8-tetrahydro-1H,3H-chinolin-2,5-dion erhielt man nach der in Beispiel 3.2 beschriebenen Methode die Titelverbindung.
ESI-MS: [M+H⁺] = 482,4, 241,7;
¹H-NMR (400 MHz, CDCl₃) δ (ppm): 6,12 (1H, s), 3,72 (2H, t), 3,62 (4H, s br.), 2,81-2,28 (16H, m; incl. 2,41 (4H, s)), 2,09 (2H, quint.), 1,70 (2H, sext.), 1,60 (4H+H₂O, s br.), 1,31 (9H, s), 0,95 (3H, t).

### Beispiel 45

### 1-{4-[4-(2-tert-Butyl-6-trifluormethyl-pyrimidin-4-yl)-piperazin-1-yl]-butyl}-4,6,7,8-tetrahydro-1H,3H-chinoline-2,5-dion

Die Herstellung erfolgte in Anlehnung an das in Beispiel 44 beschriebene Verfahren.
ESI-MS: [M+K⁺] = 546,3, [M+H⁺] = 508,3;
¹H-NMR (400 MHz, CDCl₃) δ (ppm): 6,57 (1H, s), 3,73 (6H, s br.), 2,70-2,32 (14H, m), 2,09 (2H, quint.), 1,57 (6H, s br.), 1,33 (9H, s).

### Beispiel 46

### 1-{4-[4-(2-tert-Butyl-6-propylpyrimidin-4-yl)piperazin-1-yl]butyl}-1,3,4,5-tetrahydro-2H-1-benzazepin-2-on

### 46.1 Gemisch aus 1-(4-Chlorbutyl)-1,3,4,5-tetrahydro-2H-1-benzazepin-2-on und 1-(4-Brombutyl)-1,3,4,5-tetrahydro-2H-1-benzazepin-2-on

Ausgehend von 1,3,4,5-Tetrahydro-2H-1-benzazepin-2-on (6,20 mmol, 1,00 g; hergestellt wie in J. Heterocycl. Chem. 1983, 20, 663-6) und 1-Brom-4-chlorbutan (7,44 mmol, 1,28 g) erhielt man nach der in Beispiel 1 beschriebenen Methode 1,53 g des Gemischs aus 1-(4-Chlorbutyl)-1,3,4,5-tetrahydro-2H-1-benzazepin-2-on und 1-(4-Brombutyl)-1,3,4,5-tetrahydro-2H-1-benzazepin-2-on als etwa 1:1-Gemisch.
ESI-MS (Cl-Verbindung): [M+H+] = 252,1;
ESI-MS (Br-Verbindung): [M+H+] = 296,0.

### 46.2 1-{4-[4-(2-tert-Butyl-6-propylpyrimidin-4-yl)piperazin-1-yl]butyl}-1,3,4,5-tetrahydro-2H-1-benzazepin-2-on

Ausgehend von dem Gemisch aus 1-(4-Chlorbutyl}-1,3,4,5-tetrahydro-2H-1-benzazepin-2-on und 1-(4-Brombutyl)-1,3,4,5-tetrahydro-2H-1-benzazepin-2-on (2,86 mmol, 0,80 g) und 2-tert-Butyl-4-piperazin-1-yl-6-propylpyrimidin (2,86 mmol, 0,75 g; hergestellt wie in DE 19735410) erhielt man nach der in Beispiel 3.2 beschriebenen Methode 0,49 g der Titelverbindung.
ESI-MS: [M+H⁺] = 478.4, 239.7.

### Beispiel 47

### 5-{4-[4-(2-tert-Butyl-6-propylpyrimidin-4-yl)piperazin-1-yl]butyl}-2,3-dihydro-1,5-benzothiazepin-4(5H)-on

### 47.1 5-(4-Chlorbutyl)-2,3-dihydro-1,5-benzothiazepin-4(5H)-on

Ausgehend von 2,3-Dihydro-1,5-benzothiazepin-4(5*H*)-on (27,90 mmol, 5,00 g; hergestellt wie in DE 3800386) und 1-Brom-4-chlorbutan (33,47 mmol, 5,74 g) erhielt man nach der in Beispiel 1 beschriebenen Methode 4,60 g der Titelverbindung.
ESI-MS: [M+H⁺] = 270,0.

### 47.2 5-{4-[4-(2-tert-Butyl-6-propylpyrimidin-4-yl)piperazin-1-yl]butyl}-2,3-dihydro-1,5-benzothiazepin-4(5H)-on

Ausgehend von 5-(4-Chlorbutyl)-2,3-dihydro-1,5-benzothiazepin-4(5*H*)-on (2,67 mmol, 0,80 g) und 2-*tert*-Butyl-4-piperazin-1-yl-6-propylpyrimidin (2,67 mmol, 0,70 g; hergestellt wie in DE 19735410) erhielt man nach der in Beispiel 3.2 beschriebenen Methode 0,58 g der Titelverbindung.
ESI-MS: [M+H+] = 496,4, 248,7.

### Beispiel 48

### 1-(5-{4-[2-tert-Butyl-6-(trifluormethyl)pyrimidin-4-yl]piperazin-1-yl}pentanoyl)-2,3,4,5-tetrahydro-1H-1-benzazepin

### 48.1 1-(5-Chlorpentanoyl)-2,3,4,5-tetrahydro-1H-1-benzazepin

Ausgehend von 2,3,4,5-Tetrahydro-1*H*-1-benzazepin (16,13 mmol, 2,50 g; hergestellt wie in Org. Lett. 2002, 4, 261-4) und 5-Chlorvaleriansäurechlorid (24,20 mmol, 3,75 g) erhielt man nach der in Beispiel 18.1 beschriebenen Methode 4,25 g der Titelverbindung.
ESI-MS: 269,1, 268,1, 266,1.

### 48.2 1-(5-{4-[2-tert-Butyl-6-(trifluormethyl)pyrimidin-4-yl]piperazin-1-yl}pentanoyl)-2,3,4,5-tetrahydro-1H-1-benzazepin

Ausgehend von 1-(5-Chlorpentanoyl)-2,3,4,5-tetrahydro-1*H*-1-benzazepin (1,88 mmol, 0,50 g) erhielt man nach der in Beispiel 3.2 beschriebenen Methode 0,67 g der Titelverbindung.
ESI-MS: [M+H⁺] = 518,2, 259,8.

### Beispiel 49

### 1-{5-[4-(2-tert-Butyl-6-propylpyrimidin-4-yl)piperazin-1-yl]pentanoyl}-2,3,4,5-tetrahydro-1H-1-benzazepin

Ausgehend von 1-(5-Chlorpentanoyl)-2,3,4,5-tetrahydro-1*H*-1-benzazepin aus Beispiel 48.1 (1,88 mmol, 0,50 g) und 2-*tert*-Butyl-4-piperazin-1-yl-6-propylpyrimidin (1,88 mmol, 0,49 g; hergestellt wie in DE 19735410) erhielt man nach der in Beispiel 3.2 beschriebenen Methode 0,60 g der Titelverbindung.
ESI-MS: [M+H⁺] = 492,7, 246,97;

### Beispiel 50

### 1-(4-{4-[2-tert-Butyl-6-(trifluormethyl)pyrimidin-4-yl]piperazin-1-yl}butyl)-1,3,4,5-tetrahydro-2H-1-benzazepin-2-on

Man erwärmte ein Gemisch aus 1-(4-Chlorbutyl)-1,3,4,5-tetrahydro-2*H*-1-benzazepin-2-on und 1-(4-Brombutyl)-1,3,4,5-tetrahydro-2*H*-1-benzazepin-2-on (0,40 g), 2-tert-Butyl-4-piperazin-1-yl-6-(trifluormethyl)pyrimidin (1,59 mmol, 0,46 g, hergestellt wie in DE 19735410), NaBr (7,94 mmol, 0,82 g), Diisopropylethylamin (15,57 mmol, 2,01 g) und N-Methylpyrrolidinon (0,6 ml) 5 Stunden auf 120 °C. Anschließend filtrierte man die Suspension und engte das Filtrat bis zur Trockne ein. Den Rückstand nahm man in Essigsäureethylester auf und extrahierte mit einer gesättigten Kochsalzlösung. Die organische Phase trocknete man, filtrierte das Trockenmittel ab und engte bis zur Trockne ein. Den Rückstand reinigte man durch Chromatographie an Kieselgel (Eluierungsmittel: Dichlormethan/MeOH (0-100%), wobei man 0,58 g der Titelverbindung erhielt.
ESI-MS: [M+H⁺] = 504,4, 252,7.

### Beispiel 51

### 5-(4-{4-[2-tert-Butyl-6-(trifluormethyl)pyrimidin-4-yl]piperazin-1-yl}butyl)-2,3-dihydro-1,5-benzothiazepin-4(5H)-on

Ausgehend von 5-(4-Chlorbutyl)-2,3-dihydro-1,5-benzothiazepin-4(5*H*)-on aus Beispiel 47.1 (2,79 mmol, 0,50 g) erhielt man nach der in Beispiel 50 beschriebenen Methode 0,57 g der Titelverbindung.
ESI-MS: [M+H⁺] = 522,2, 261,6.

### Beispiel 52

### 4-[4-(2-tert-Butyl-6-trifluormethyl-pyrimidin-4-yl)-piperazin-1-yl]-1-(2,3,4,5-tetrahydro-benzo[b]azepin-1-yl)-butan-1-on

### 52.1 4-Chlor-1-(2,3,4,5-tetrahydro-benzo[b]azepin-1-yl)-butan-1-on

Ausgehend von 2,3,4,5-Tetrahydro-1*H*-1-benzazepin (3,40 mmol, 0,50 g; hergestellt wie in Org. Lett. 2002, 4, 261-4) und 5-Chlorbuttersäurechlorid (5,09 mmol, 0,73 g) erhielt man nach der in Beispiel 18.1 beschriebenen Methode 0,80 g der Titelverbindung.
ESI-MS: [M+H⁺] = 252,1.

### 52.2 4-[4-(2-tert-Butyl-6-trifluormethyl-pyrimidin-4-yl)-piperazin-1-yl]-1-(2,3,4,5-tetrahydro-benzo[b]azepin-1-yl)-butan-1-on

Ausgehend von 4-Chlor-1-(2,3,4,5-tetrahydro-benzo[b]azepin-1-yl)-butan-1-on (1,19 mmol, 0,30 g) erhielt man nach der in Beispiel 3.2 beschriebenen Methode 0,42 g der Titelverbindung.
ESI-MS: [M+H⁺] = 504,4, 252,6.

### Beispiel 53

### 4-[4-(2-tert-Butyl-6-propyl-pyrimidin-4-yl)-piperazin-1-yl]-1-(2,3,4,5-tetrahydro-benzo[b]azepin-1-yl)-butan-1-on

Ausgehend von 4-Chlor-1-(2,3,4,5-tetrahydro-benzo[b]azepin-1-yl)-butan-1-on aus Beispiel 52.1 (1,19 mmol, 0,30 g) und 2-tert-Butyl-4-piperazin-1-yl-6-propylpyrimidin (1.19 mmol, 0,31 g; hergestellt wie in DE 19735410) erhielt man nach der in Beispiel 3.2 beschriebenen Methode 0,36 g der Titelverbindung.
ESI-MS: [M+H⁺] = 478,4.

In analoger Weise wurden die Verbindungen der Beispiele 54 bis 57 hergestellt:

### Beispiel 54:

### 1-{4-[4-(2-tert-Butyl-6-propyl-pyrimidin-4-yl)-piperain-1-yl]-butyl}-6-methoxy-1,3,4,5-tetrahydro-benzo[b]azepin-2-on

ESI-MS: [M+Na⁺] = 530,5, 509,5, [M+H⁺] = 508,5, 254,9;
¹H-NMR (400 MHz, DMSO-d₆) δ (ppm): 7,28 (1H, t), 6,96 (1H, d), 6,89 (1H, d), 6,41 (1H, s), 3,80 (3H, s), 3,53 (4H, s br.), 2,45 (2H, t), 2,31 (4H, s br.), 2,19 (2H, t), 2,06 (2H, s), 1,63 (2H, sext.), 1,54-1,32 (4H, m), 1,25 (9H, s), 0,89 (3H, t).

### Beispiel 55:

### 4-(2-tert-Butyl-6-trifluormethylpyrimidin-4-yl)-1-[4-(6-methoxy-2-oxo-2,3,4,5-tetrahydro-benzo[b]azepin-1-yl)-butyl]-piperazin als Fumarat

ESI-MS: [M+H⁺] = 534,2, 267,6;

### Beispiel 56

### 4-(2-tert-butyl-6-propyl-pyrimidin-4-yl)-1-[4-(6-hydroxy-2-oxo-2,3,4,5-tetrahydro-benzo[b]azepin-1-yl)-butyl]-piperazin als Fumarat

ESI-MS: [M+H⁺] = 494,5, 247,7;
¹H-NMR (400 MHz, DMSO-d₆) δ (ppm): 9,55 (1H, s), 7,09 (1H. t), 6,79 (1H, d), 6,71 (1H, d), 6,43 (1H, s), 3,54 (4H, s br.), 2,46 (2H, t), 2,36 (4H, m), 2,25 (2H, t), 2,08 (2H, s br.), 1,63 (2H, sext.), 1,39 (4H, m), 1,25 (9H, s), 0,90 (3H, t).

### Beispiel 57

### 4-(2-tert-Butyl-6-trifluoromethyl-pyrimidin-4-yl)-1-[4-(6-hydroxy-2-oxo-2,3,4,5-tetrahydro-benzo[b]azepin-1-yl)-butyl]-piperazin als Fumarat

ESI-MS: [M+Na⁺] = 542,3, 521,3, [M+H⁺] = 520,2, 260,6;

### B) Beispiele für galenische Applikationsformen

### Tabletten:

Auf einer Tablettenpresse werden in üblicher Weise Tabletten folgender Zusammensetzung gepresst:
40 mg Substanz des Beispiels 2
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6%iger Kleister)

### Dragees:

20 mg Substanz des Beispiels 2
60 mg Kemmasse
70 mg Verzuckerungsmasse

Die Kemmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### C) Biologische Untersuchungen - Rezeptorbindungsstudien:

Die zu testende Substanz wurde entweder in Methanol/Chremophor® (BASF-AG) oder in Dimethylsulfoxid gelost und anschließend mit Wasser auf die gewünschte Konzentration verdünnt

### I. Dopamin-D₃-Rezeptor:

Der Ansatz (0,250 ml) setzte sich zusammen aus Membranen von ~ 10⁶ HEK-293 Zellen mit stabil exprimierten humanen Dopamin-D₃-Rezeptoren, 0,1 nM [¹²⁵I]-Jodosulpirid und Inkubationspuffer (totale Bindung) oder zusätzlich Testsubstanz (Hemmkurve) oder 1uM Spiperon (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1% Rinderserumalbumin, 10 µM Quinolone, 0.1 % Ascorbinsäure (täglich frisch hergestellt). Der Puffer wurde mit HCl auf pH 7,4 eingestellt.

### II. Dopamin-D2_{L}-Rezeptor:

Der Ansatz (1 ml) setzte sich zusammen aus Membranen von - 10⁶ HEK-293 Zellen mit stabil exprimierten humanen Dopamin-D_{2L} Rezeptoren (lange Isoform) sowie 0,01 nM [²⁵I]-Jodospiperon und Inkubationspuffer (totale Bindung) oder zusätzlich Testsubstanz (Hemmkurve) oder 1µM Haloperidol (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1 % Rinderserumalbumin. Der Puffer wurde mit HCl auf pH 7,4 eingestellt.

### III. Messung und Auswertung:

Nach Inkubation für 60 Minuten bei 25 °C wurden die Ansätze mit einem Zellsammelgerät über Whatman GF/B Glasfaserfilter unter Vakuum filtriert. Die Filter wurden mit einem Filter-Transfer-System in Szintillationsgläser überführt Nach Zugabe von 4 ml Ultima Gold^{®} (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschreibenen Programm "LIGAND".

Die erfindungsgemäßen Verbindungen zeigen in diesen Tests sehr gute Affinitäten am D₃-Rezeptor (< 100 nM, häufig < 50 nM und speziell < 10 nM) und binden selektiv an den D₃-Rezeptor.

Die Ergebnisse der Bindungstests sind in Tabelle 1 angegeben.

**Tabelle 1:**

| Beispiel | Kᵢ (D₃) [nM] | Selektivität vs. D₂L* |
|---|---|---|
| 1 | 3,43 | 37 |
| 2 | 12,1 | 29 |
| 10 | 9,2 | 28 |
| 33 | 1,71 | 65 |
| 36 | 1,18 | 41 |
| 37 | 1,59 | 38 |
| 44 | 1,54 | 29 |
| 45 | 4,68 | 41 |
| 54 | 2,25 | 25 |
| 56 | 0,69 | 26 |
| 57 | 2,48 | 38 |

| | | |
|---|---|---|
| * Kᵢ(D_{2L})/Kᵢ(D₃) | | |

## Patentansprüche

1. Verbindungen der allgemeinen Formel I 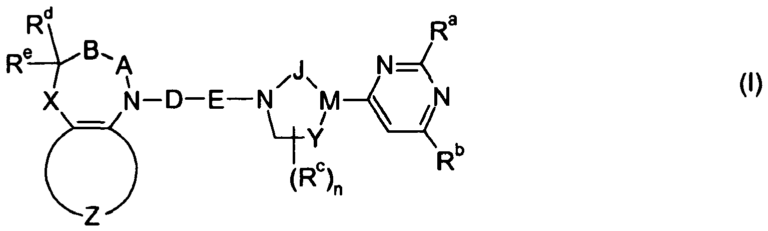 worin
A für eine Gruppe C=W oder CR^{f}R^{g} steht;
B für eine chemische Bindung oder eine Gruppe CR^{h}Rⁱ steht;
X für O, S, eine Gruppe N-R^{k} oder eine Gruppe CR^{m}Rⁿ steht;
D für C=O oder eine chemische Bindung steht;
E für eine lineare oder verzweigte 2 bis 10 gliedrige Alkylenkette steht, die als Kettenglieder 1 oder 2 nicht benachbarte Heteroatomgruppe(n) K aufweisen kann, die ausgewählt ist unter O, S, S(O), S(O)₂ und N-R^{p}, und die eine Carbonylgruppe und/oder eine Cycloalkandiyl-Gruppe umfassen kann und/oder eine Doppel- oder Dreifachbindung aufweisen kann;
W Sauerstoff oder Schwefel bedeutet;
Z gemeinsam mit den C-Atomen, an die es gebunden ist, für einen Phenylring oder einen Cyclohexenring steht, wobei Cyclohexenring eine Carbonylgruppe als Ringglied aufweisen kann und wobei der Phenylring 1, 2, 3 oder 4 Substituenten R aufweisen kann, die ausgewählt sind unter gegebenenfalls substituiertem C₁-C₆-Alkyl, CN, OR¹, NR²R³, NO₂, SR⁴, SO₂R⁴, SO₂NR²R³, CONR²R³, COOR⁵, COR⁶, C₁-C₄-Halogenalkoxy, C₂-C₆-Alkenyl, C₂-C₆-Alkinyl, C₂-C₆-Alkenyloxy, C₂-C₆-Alkinyloxy, C₃-C₆-Cycloalkyl, C₃-C₆-Cycloalkyloxy und Halogen,und/oder 2 Substituenten R gemeinsam eine Kette X'-Alk'-X" bilden können, worin X' und X" unabhängig voneinander für O oder S stehen und Alk' C₁-C₄-Alkandiyl bedeutet, das gegebenenfalls 1, 2, 3 oder 4 Alkylgruppen oder Halogenatome als Substituenten aufweist;
J für CH₂-CH₂ steht;
M für N steht;
Y für CH₂ oder CH₂-CH₂ steht;
n 0 oder 1 ist;
R^{a}, R^{b} unabhängig voneinander für C₁-C₆-Alkyl, C₁-C₆-Halogenalkyl, C₂-C₆-Alkenyl, C₃-C₆-Cycloalkyl, C₁-C₆-Alkoxy-C₁-C₄-alkyl oder C₃-₆-Cycloalkyl-C₁-C₄-alkyl stehen;
R^{c} für C₁-C₄-Alkyl steht;
R^{d}, R^{e} unabhängig voneinander ausgewählt sind unter Wasserstoff, Halogen, gegebenenfalls substituiertem C₁-C₆-Alkyl, C₃-C₆-Cycloalkyl, C₂-C₆-Alkenyl, C₂-C₆-Alkinyl, C₁-C₆-Alkoxy, C₃-C₆-Cycloalkyloxy, C₃-C₆-Cycloalkyl-C₁-C₄-alkyloxy und gegebenenfalls substituiertem Phenyl, wobei CR^{d}R^{e} gemeinsam für C=O stehen können und die Reste R^{d}, R^{e} gemeinsam eine Kette X'-Alk-X" bilden können, worin X und X" unabhängig voneinander für O oder S stehen und Alk C₂-C₄-Alkandiyl bedeutet, das gegebenenfalls 1, 2 3 oder 4 Alkylgruppen oder Halogenatome als Substituenten aufweist;
R^{f}, R^{g} unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten oder die Reste R^{f}, R^{g} gemeinsam eine Kette X'-Alk-X" bilden können, worin Alk, X' und X" die zuvor genannten Bedeutungen aufweisen;
R^{h}, Rⁱ unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten;
R^{k}, R^{p} unabhängig voneinander für Wasserstoff, C₁-C₄-Alkyl, C₁-C₆-Alkoxy, C₁-C₆-Alkoxyalkyl, C₁-C₆-Alkylcarbonyl, C₁-C₆-Alkoxycarbonyl, Phenylalkyl, Phenylcarbonyl, Phenoxycarbonyl, wobei Phenyl in den drei letzgenannten Gruppen 1, 2 oder 3 Substituenten tragen kann, die ausgewählt sind unter C₁-C₄-Alkyl, C₁-C₄-Alkoxy, NR²R³, CN, C₁-C₂-Fluoralkyl und Halogen;
R^{m}, Rⁿ unabhängig voneinander Wasserstoff, Halogen, gegebenenfalls substituiertes C₁-C₆-Alkyl, C₁-C₆-Alkoxy, C₂-C₆-Alkenyl, C₂-C₆-Alkinyl, C₃-C₆-Cycloalkyloxy, C₃-C₆-Cycloalkyl-C₁-C₄-alkyloxy oder C₃-C₆-Cycloalkyl bedeuten; und
wobei, wenn X für CR^{m}Rⁿ oder N-R^{k} steht, einer der Reste R^{d} oder R^{e} gemeinsam mit einem der Reste R^{m}, Rⁿ oder R^{k} auch eine π-Bindung bedeuten können;
R¹, R², R³, R⁴, R⁵ und R⁶ unabhängig voneinander für H, gegebenenfalls substituiertes C₁-C₆-Alkyl oder gegebenenfalls substituiertes Phenyl, stehen, wobei R³ auch eine Gruppe COR⁷ bedeuten kann, wobei R⁷ für Wasserstoff, gegebenenfalls substituiertes C₁-C₄-Alkyl oder gegebenenfalls substituiertes Phenyl steht, wobei R² mit R³ auch gemeinsam mit dem Stickstoffatom, an das sie gebunden sind, einen 5- oder 6-gliedrigen, gesättigten oder ungesättigten Heterocyclus bilden können, der ein weiteres Heteroatom, ausgewählt unter O, S und NR³ als Ringglied aufweisen kann, wobei R⁸ für Wasserstoff oder C₁-C₄-Alkyl steht, und
die physiologisch akzeptablen Säureadditionssalze dieser Verbindungen.

2. Verbindungen der allgemeinen Formel I nach Anspruch 1, worin Z gemeinsam mit den C-Atomen, an die es gebunden ist, für einen kondensierten Phenylring steht, der 1, 2, 3 oder 4 der zuvor genannten Substituenten R aufweisen kann.

3. Verbindungen der allgemeinen Formel I nach einem der vorhergehenden Ansprüche, worin entweder A für eine Gruppe C=W oder D für C=O steht.

4. Verbindungen der allgemeinen Formel I nach einem der vorhergehenden Ansprüche, worin E für eine Gruppe (CH₂)ₖ bedeutet, worin k für 3, 4, 5 oder 6 steht.

5. Verbindungen der allgemeinen Formel I nach einem der vorhergehenden Ansprüche, worin B eine chemische Bindung bedeutet.

6. Verbindungen der allgemeinen Formel I nach einem der Ansprüche 1 bis 4, worin B für CH₂ steht.

7. Verbindungen der allgemeinen Formel I nach einem der vorhergehenden Ansprüche, worin J für CH₂-CH₂ und Y für CH₂ stehen.

8. Verbindungen der allgemeinen Formel I nach Anspruch 1, worin R^{a} für C₁-C₆-Alkyl steht und R^{b} ausgewählt ist unter C₁-C₆-Alkyl, C₃-C₆-Cycloalkyl und C₁-C₂-Fluoralkyl.

9. Verbindungen nach einem der vorhergehenden Ansprüche der allgemeinen Formel Ia, 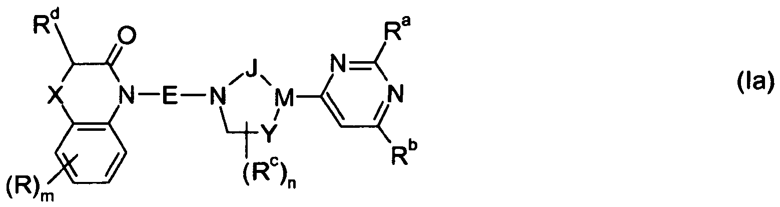 worin n, X, E, J, M, R, R^{a}, R^{b}, R^{c} und R^{d} die zuvor genannten Bedeutungen aufweisen und m für 0, 1, 2 oder 3 steht.

10. Verbindungen nach einem der Ansprüche 1 bis 8 der allgemeinen Formel Ib, 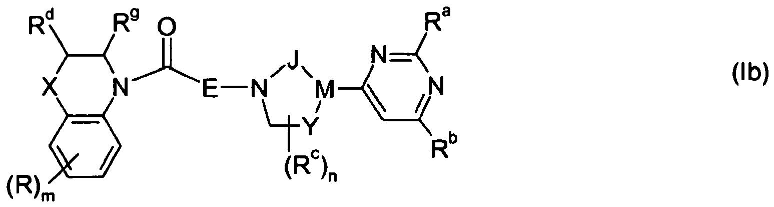 worin n, X, E, J, M, R, R^{a}, R^{b}, R^{c}, R^{d} und R^{g} die zuvor genannten Bedeutungen aufweisen und m für 0, 1, 2 oder 3 steht.

11. Verbindungen nach einem der Ansprüche 1 bis 8 der allgemeinen Formel Ic, 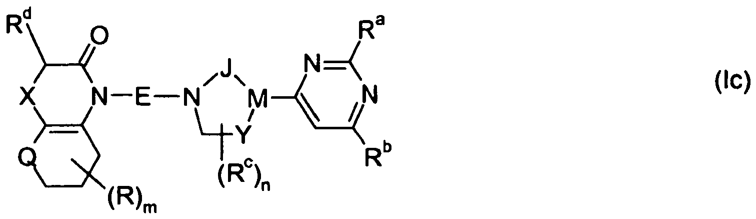 worin n, X, E, J, M, R, R^{a}, R^{b}, R^{c} und R^{d} die zuvor genannten Bedeutungen aufweisen, Q für CH₂ oder C=O steht und m für 0 steht.

12. Verbindungen nach einem der Ansprüche 1 bis 8 der allgemeinen Formel Id, 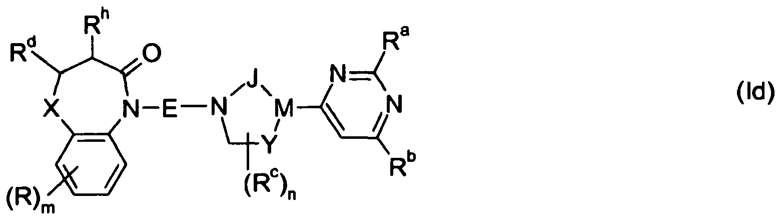 worin n, X, E, J, M, R, R^{a}, R^{b}, R^{c}, R^{d} und R^{h} die zuvor genannten Bedeutungen aufweisen und m für 0, 1, 2 oder 3 steht.

13. Verbindungen nach einem der Ansprüche 1 bis 8 der allgemeinen Formel Ie, 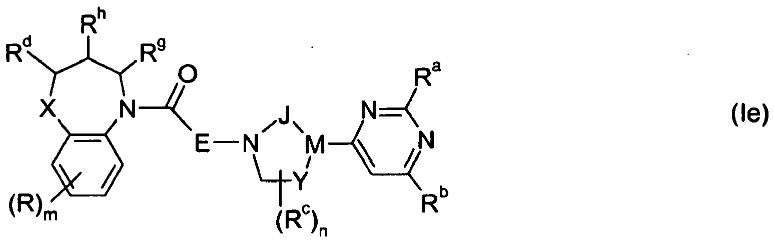 worin n, X, E, J, M, R, R^{a}, R^{b}, R^{c}, R^{d}, R^{g} und R^{h} die zuvor genannten Bedeutungen aufweisen und m für 0, 1, 2 oder 3 steht.

14. Pharmazeutisches Mittel, enthaltend wenigstens einen Wirkstoff, der ausgewählt ist unter Verbindungen der Formel I und den physiologisch verträglichen Säureadditionssalzen der Verbindungen I gemäß einem der Ansprüche 1 bis 13, gegebenenfalls zusammen mit physiologisch akzeptablen Trägern und/oder Hiffsstoffen.

15. Verwendung von Wirkstoffen, die ausgewählt sind unter Verbindungen der Formel I und den physiologisch verträglichen Säureadditionssalzen der Verbindungen **I** gemäß einem der Ansprüche 1 bis 13, zur Herstellung eines pharmazeutischen Mittels zur Behandlung von Erkrankungen und Störungen, ausgewählt aus Störungen und Erkrankungen des zentralen Nervensystems und Nierenfunktionsstörungen, affektiven Störungen, neurotischen Störungen, Belastungs- und somatoformen Störungen und Psychosen, speziell Schizo- phrenie und Depression und weiterhin Nierenfunktionsstörungen, insbesondere Nieren- funktionsstörungen, die durch Diabetes mellitus hervorgerufen werden, die auf die Beeinflussung durch Dopamin-D₃-Rezeptorantagonisten bzw. -agonisten ansprechen.

16. Verwendung nach Anspruch 15 zur Herstellung eines pharmazeutischen Mittels zur Behandlung von Erkrankungen des zentralen Nervensystems.

17. Verwendung nach Anspruch 16 zur Herstellung eines pharmazeutischen Mittels zur Behandlung von Schizophrenie und/oder Depression.

## Claims

1. Compounds of the general formula I 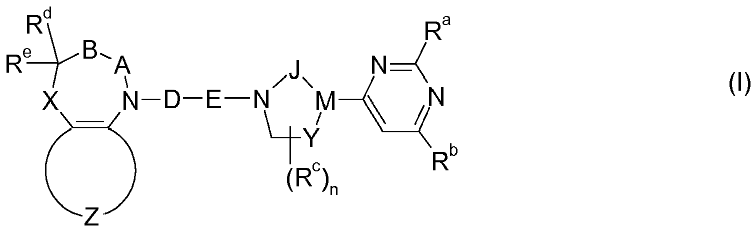 in which
A is a C=W or CR^{f}R^{g} group;
B is a chemical bond or a CR^{h}Rⁱ group;
X is O, S, an N-R^{k} group or a CR^{m}Rⁿ group;
D is C=O or a chemical bond;
E is a linear or branched 2 to 10 membered alkylene chain which may have as chain members 1 or 2 non-adjacent heteroatomic group(s) K which is selected from O, S, S(O), S(O)₂ and N-R^{p}, and which may comprise a carbonyl group and/or a cycloalkanediyl group and/or may have a double or triple bond;
W is oxygen or sulfur;
Z is together with the C atoms to which it is bonded a phenyl ring or a cyclohexene ring, where the cyclohexene ring may have a carbonyl group as ring member and where the phenyl ring may have 1, 2, 3 or 4 substituents R which are selected from optionally substituted C₁-C₆-alkyl, CN, OR¹, NR²R³, NO₂, SR⁴,SO₂R⁴, So₂NR²R³, CONR²R³, COOR⁵, COR⁶, C₁-C₄-haloalkoxy, C₂-C₆-alkenyl, C₂-C₆-alkynyl, C₂-C₆-alkenyloxy, C₂-C₆-alkynyloxy, C₃-C₆-cycloalkyl, C₃-C₆-cycloalkyloxy and halogen and/or 2 substituents R may together form a chain X'-Alk'-X" in which X' and X" are independently of one another O or S, and Alk' is C₁-C₄-alkanediyl which optionally has 1, 2, 3 or 4 alkyl groups or halogen atoms as substituents;
J is CH₂-CH₂;
M is N;
Y is CH₂ or CH₂-CH_{2;}
n is 0 or 1;
R^{a}, R^{b} are I ndependently of one another C₁-C₆-alkyl, C₁-C₆-haloalkyl, C₂-C₆-alkenyl, C₃-C₆-cycloalkyl, C₁-C₆-alkoxy-C₁-C₄-alkyl or C₃-C₆-cycloalkyl-C₁-C₄-alkyl;
R^{c} is C₁-C₄-alkyl;
R^{d}, R^{e} are independently of one another selected from hydrogen, halogen, optionally substituted C₁-C₆-alkyl, C₃-C₆-cycloalkyl, C₂-C₆-alkenyl, C₂-C₆-alkynyl, C₁-C₆-alkoxy, C₃-C₆-cycloalkyloxy, C₃-C₆-cycloalkyl-C₁-C₄-alkyloxy and optionally substituted phenyl, where CR^{d}R^{e} may together be C=O, and the radicals R^{d}, R^{e} may together form a chain X'-Alk-X" in which X' and X" are independently of one another O or S, and Alk is C₂-C₄-alkanediyl which optionally has 1, 2 3 or 4 alkyl groups or halogen atoms as substituents;
R^{f}, R^{g} are independently of one another hydrogen, halogen, optionally substituted C₁-C₆-alkyl, C₁-C₆-alkoxy, C₂-C₆-alkenyl, C₂-C₆-alkynyl, C₃-C₆-cycloalkyloxy, C₃-C₆-cycloalkyl-C₁-C₄-alkyloxy or C₃-C₆-cycloalkyl, or the radicals R^{f}, R^{g} may together form a chain X'-Alk-X" in which Alk, X' and X" have the aforementioned meanings;
R^{h}, Rⁱ are independently of one another hydrogen, halogen, optionally substituted C₁-C₆-alkyl, C₁-C₆-alkoxy, C₂-C₆-alkenyl, C₂-C₆-alkynyl, C₃-C₆-cycloalkyloxy, C₃-C₆-cycloalkyl-C₁-C₄-alkyloxy or C₃-C₆-cycloalkyl;
R^{k}, R^{p} are independently of one another hydrogen, C₁-C₄-alkyl, C₁-C₆-alkoxy, C₁-C₆-alkoxyalkyl, C₁-C₆-alkylcarbonyl, C₁-C₆-alkoxycarbonyl, phenylalkyl, phenylcarbonyl, phenoxycarbonyl, where phenyl in the last three groups mentioned may have 1, 2 or 3 substituents which are selected from C₁-C₄-alkyl, C₁-C₄-alkoxy, NR²R³, CN, C₁-C₂-fluoroalkyl and halogen;
R^{m}, Rⁿ are independently of one another hydrogen, halogen, optionally substituted C₁-C₆-alkyl, C₁-C₆-alkoxy, C₂-C₆-alkenyl, C₂-C₆-alkynyl, C₃-C₆-cycloalkyloxy, C₃-C₆-cycloalkyl-C₁-C₄-alkyloxy or C₃-C₆-cycloalkyl; and
in the case where X is CR^{m}Rⁿ or N-R^{k}, one of the radicals R^{d} or R^{e} may together with one of the radicals R^{m}, Rⁿ or R^{k} also be a π bond;
R¹, R², R³, R⁴, R⁵ and R⁶ are independently of one another H, optionally substituted C₁-C₆-alkyl or optionally substituted phenyl, where R³ may also be a group COR⁷, where R⁷ is hydrogen, optionally substituted C₁-C₄-alkyl or optionally substituted phenyl, where R² with R³ may also form together with the nitrogen atom to which they are bonded a 5- or 6-membered, saturated or unsaturated heterocycle which may have a further heteroatom selected from O, S and NR⁸ as ring member, where R⁸ is hydrogen or C₁-C₄-alkyl, and
the physiologically acceptable acid addition salts of these compounds.

2. Compounds of the general formula I according to Claim 1, in which Z together with the C atoms to which it is bonded stands for a fused phenyl ring which may have 1, 2, 3 or 4 of the aforementioned substituents R.

3. Compounds of the general formula I according to either of the preceding claims, in which either A is a C=W group or D is C=O.

4. Compounds of the general formula I according to any of the preceding claims, in which E is a (CH₂)ₖ group in which k is 3, 4, 5 or 6.

5. Compounds of the general formula I according to any of the preceding claims, in which B is a chemical bond.

6. Compounds of the general formula I according to any of Claims 1 to 4, in which B is CH₂.

7. Compounds of the general formula I according to any of the preceding claims, in which J is CH₂-CH₂ and Y is CH₂.

8. Compounds of the general formula I according to Claim 1, in which R^{a} is C₁-C₆-alkyl, and R^{b} is selected from C₁-C₆-alkyl, C₃-C₆-cycloalkyl and C₁-C₂-fluoroalkyl.

9. Compounds according to any of the preceding claims of the general formula la, 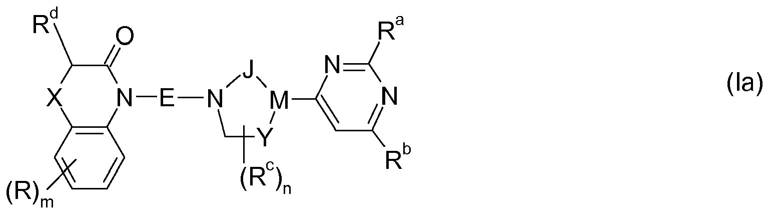 in which n, X, E, J, M, R, R^{a}, R^{b}, R^{c} and R^{d} have the aforementioned meanings, and m is 0, 1, 2 or 3.

10. Compounds according to any of Claims 1 to 8 of the general formula Ib, 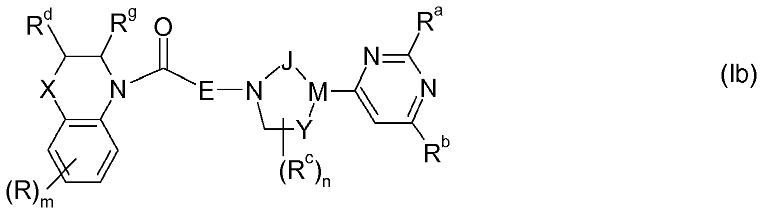 in which n, X, E, J, M, R, R^{a}, R^{b}, R^{c}, R^{d} and R^{g} have the aforementioned meanings, and m is 0, 1, 2 or 3.

11. Compounds according to any of Claims 1 to 8 of the general formula Ic, 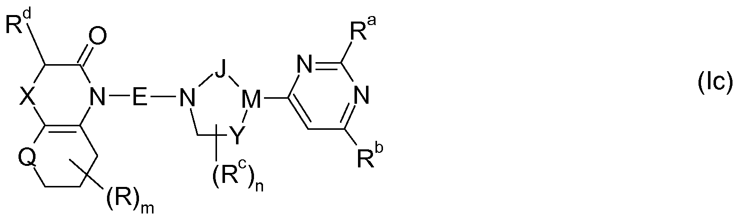 in which n, X, E, J, M, R, R^{a}, R^{b}, R^{c} and R^{d} have the aforementioned meanings, Q is CH₂ or C=O, and m is 0.

12. Compounds according to any of Claims 1 to 8 of the general formula Id, 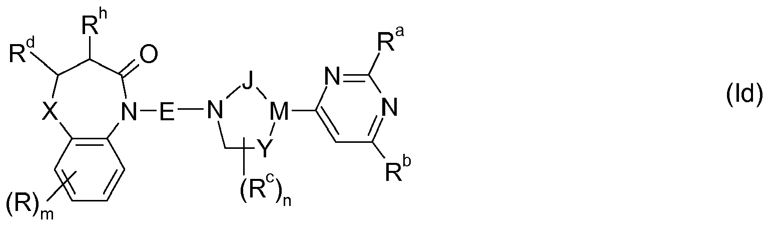 in which n, X, E, J, M, R, R^{a}, R^{b}, R^{c}, R^{d} and R^{h} have the aforementioned meanings, and m is 0, 1, 2 or 3.

13. Compounds according to any of Claims 1 to 8 of the general formula Ie, 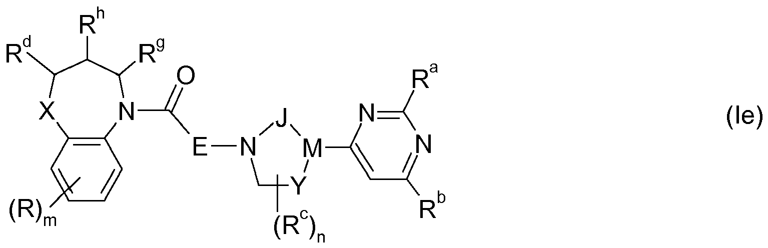 in which n, X, E, J, M, R, R^{a}, R^{b}, R^{c}, R^{d}, R^{g} and R^{h} have the aforementioned meanings, and m is 0, 1, 2 or 3.

14. Pharmaceutical composition comprising at least one active ingredient which is selected from compounds of the formula I and the physiologically tolerated acid addition salts of the compounds I according to any of Claims 1 to 13, if appropriate together with physiologically acceptable carriers and/or excipients.

15. Use of active ingredients selected from compounds of the formula I and the physiologically tolerated acid addition salts of the compounds I according to any of Claims 1 to 13 for producing a pharmaceutical composition for the treatment of conditions and disorders selected from disorders and conditions of the central nervous system and renal function disorders, affective disorders, neurotic disorders, stress disorders and somatoform disorders and psychoses, specifically schizophrenia and depression and furthermore renal function disorders, especially renal function disorders caused by diabetes mellitus, which respond to influencing by dopamine D₃ receptor antagonists or agonists.

16. Use according to Claim 15 for producing a pharmaceutical composition for the treatment of conditions of the central nervous system.

17. Use according to Claim 16 for producing a pharmaceutical composition for the treatment of schizophrenia and/or depression.

## Revendications

1. Composés de formule générale I 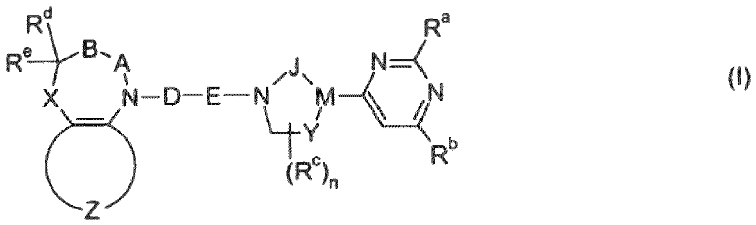 dans laquelle
A représente un groupe C=W ou CR^{f}R^{g} ;
B représente une liaison chimique ou un groupe CR^{h}Rⁱ ;
X représente 0, S, un groupe N-R^{k} ou un groupe CR^{m}Rⁿ ;
D représente C=O ou une liaison chimique ;
E représente une chaîne alkylène linéaire ou ramifiée de 2 à 10 éléments, qui peut comprendre en tant qu'éléments de chaîne 1 ou 2 groupes d'hétéroatomes non voisins K, choisis parmi 0, S, S(O), S(O)2 et N-R^{p}, et qui peut comprendre un groupe carbonyle et/ou un groupe cycloalcanediyle et/ou qui peut comprendre une double ou triple liaison ;
W signifie oxygène ou soufre ;
Z représente, conjointement avec les atomes C auxquels il est relié, un cycle phényle ou un cycle cyclohexène, le cycle cyclohexène pouvant comprendre un groupe carbonyle en tant qu'élément de cycle et le cycle phényle pouvant comprendre 1, 2, 3 ou 4 substituants R, choisis parmi alkyle en C₁-C₆ éventuellement substitué, CN, OR¹, NR²R³, NO₂, SR⁴, SO₂R⁴, SO₂NR²R³, CONR²R³, COOR⁵, COR⁶, halogénoalcoxy en C₁-C₄, alcényle en C₂-C₆, alcynyle en C₂-C₆, alcényloxy en C₂-C₆, alcynyloxy en C₂-C₆, cycloalkyle en C₃-C₆, cycloalkyloxy en C₃-C₆ et halogène, et/ou 2 substituants R pouvant former ensemble une chaîne X'-Alk'-X", X' et X" représentant indépendamment l'un de l'autre 0 ou S et Alk' signifiant alcanediyle en C₁-C₄, qui comprend éventuellement 1, 2, 3 ou 4 groupes alkyle ou atomes d'halogène en tant que substituants ;
J représente CH₂-CH₂ ;
M représente N ;
Y représente CH₂ ou CH₂-CH₂ ;
n représente 0 ou 1 ;
R^{a}, R^{b} représentent indépendamment l'un de l'autre alkyle en C₁-C₆, halogénoalkyle en C₁-C₆, alcényle en C₂-C₆, cycloalkyle en C₃-C₆, alcoxy en C₁-C₆-alkyle en C₁-C₄ ou cycloalkyle en C₃-C₆-alkyle en C₁-C₄ ;
R^{c} représente alkyle en C₁-C₄ ;
R^{d}, R^{e} sont choisis indépendamment l'un de l'autre parmi hydrogène, halogène, alkyle en C₁-C₆ éventuellement substitué, cycloalkyle en C₃-C₆, alcényle en C₂-C₆, alcynyle en C₂-C₆, alcoxy en C₁-C₆, cycloalkyloxy en C₃-C₆, cycloalkyle en C₃-C₆-alkyloxy en C₁-C₄ et phényle éventuellement substitué, CR^{d}R^{e} pouvant représenter ensemble C=O et les radicaux R^{d}, R^{e} pouvant former ensemble une chaîne X'-Alk-X", X' et X" représentant indépendamment l'un de l'autre 0 ou S et Alk signifiant alcanediyle en C₂-C₄, qui comprend éventuellement 1, 2, 3 ou 4 groupes alkyle ou atomes d'halogène en tant que substituants ;
R^{f}, R^{g} signifient indépendamment l'un de l'autre hydrogène, halogène, alkyle en C₁-C₆ éventuellement substitué, alcoxy en C₁-C₆, alcényle en C₂-C₆, alcynyle en C₂-C₆, cycloalkyloxy en C₃-C₆, cycloalkyle en C₃-C₆-alkyloxy en C₁-C₄ ou cycloalkyle en C₃-C₆, ou les radicaux R^{t}, R^{g} peuvent former ensemble une chaîne X'-Alk-X", Alk, X' et X" présentant les significations indiquées précédemment ;
R^{h}, Rⁱ signifient indépendamment l'un de l'autre hydrogène, halogène, alkyle en C₁-C₆ éventuellement substitué, alcoxy en C₁-C₆, alcényle en C₂-C₆, alcynyle en C₂-C₆, cycloalkyloxy en C₃-C₆, cycloalkyle en C₃-C₆-alkyloxy en C₁-C₄ ou cycloalkyle en C₃-C₆ ;
R^{k}, R^{p} signifient indépendamment l'un de l'autre hydrogène, alkyle en C₁-C₄, alcoxy en C₁-C₆, alcoxyalkyle en C₁-C₆, alkylcarbonyle en C₁-C₆, alcoxycarbonyle en C₁-C₆, phénylalkyle, phénylcarbonyle, phénoxycarbonyle, phényle dans les trois derniers groupes cités pouvant porter 1, 2 ou 3 substituants choisis parmi alkyle en C₁-C₄, alcoxy en C₁-C₄, NR²R³, CN, fluoroalkyle en C₁-C₂ et halogène ;
R^{m}, Rⁿ signifient indépendamment l'un de l'autre hydrogène, halogène, alkyle en C₁-C₆ éventuellement substitué, alcoxy en C₁-C₆, alcényle en C₂-C₆, alcynyle en C₂-C₆, cycloalkyloxy en C₃-C₆, cycloalkyle en C₃-C₆-alkyloxy en C₁-C₄ ou cycloalkyle en C₃-C₆ ; et
lorsque X représente CR^{m}Rⁿ ou N-R^{k}, un des radicaux R^{d} ou R^{e} peut également signifier conjointement avec un des radicaux R^{m}, Rⁿ ou R^{k} une liaison Π ;
R¹, R², R³, R⁴, R⁵ et R⁶ représentent indépendamment les uns des autres H, alkyle en C₁-C₆ éventuellement substitué ou phényle éventuellement substitué, R³ pouvant également signifier un groupe COR⁷, R⁷ représentant hydrogène, alkyle en C₁-C₄ éventuellement substitué ou phényle éventuellement substitué, R² pouvant également former avec R³, conjointement avec l'atome d'azote auquel ils sont reliés, un hétérocycle saturé ou insaturé de 5 ou 6 éléments, qui peut comprendre un hétéroatome supplémentaire choisi parmi 0, S et NR⁸ en tant qu'élément de cycle, R⁸ représentant hydrogène ou alkyle en C₁-C₄, et
les sels d'addition acide physiologiquement acceptables de ces composés.

2. Composés de formule générale I selon la revendication 1, dans lesquels Z représente conjointement avec les atomes C auxquels il est relié un cycle phényle condensé qui peut comprendre 1, 2, 3 ou 4 des substituants R indiqués précédemment.

3. Composés de formule générale I selon l'une quelconque des revendications précédentes, dans lesquels A représente un groupe C=W ou D représente C=O.

4. Composés de formule générale I selon l'une quelconque des revendications précédentes, dans lesquels E signifie un groupe (CH₂)ₖ, k représentant 3, 4, 5 ou 6.

5. Composés de formule générale I selon l'une quelconque des revendications précédentes, dans lesquels B signifie une liaison chimique.

6. Composés de formule générale I selon l'une quelconque des revendications 1 à 4, dans lesquels B représente CH₂.

7. Composés de formule générale I selon l'une quelconque des revendications précédentes, dans lesquels J représente CH₂-CH₂ et Y représente CH₂.

8. Composés de formule générale I selon la revendication 1, dans lesquels R^{a} représente alkyle en C₁-C₆ et R^{b} est choisi parmi alkyle en C₁-C₆, cycloalkyle en C₃-C₆ et fluoroalkyle en C₁-C₂.

9. Composés selon l'une quelconque des revendications précédentes, de formule générale la 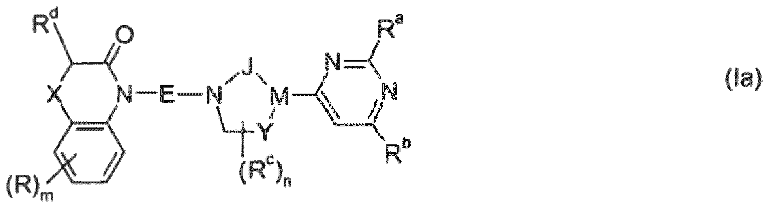 dans laquelle n, X, E, J, M, R, R^{a}, R^{b}, R^{c} et R^{d} présentent les significations indiquées précédemment et m représente 0, 1, 2 ou 3.

10. Composés selon l'une quelconque des revendications 1 à 8, de formule générale Ib 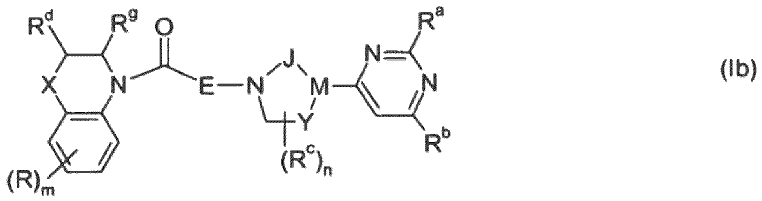 dans laquelle n, X, E, J, M, R, R^{a}, R^{b}, R^{c}, R^{d} et R^{g} présentent les significations indiquées précédemment et m représente 0, 1, 2 ou 3.

11. Composés selon l'une quelconque des revendications 1 à 8, de formule générale Ic 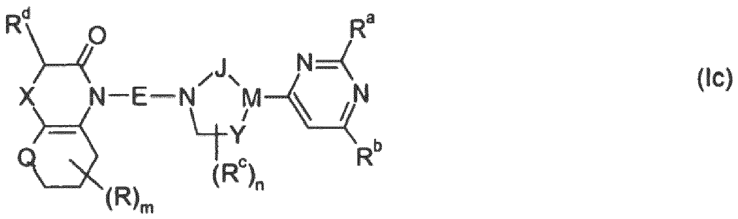 dans laquelle n, X, E, J, M, R, R^{a}, R^{b}, R^{c} et R^{d} présentent les significations indiquées précédemment, Q représente CH₂ ou C=O et m représente 0.

12. Composés selon l'une quelconque des revendications 1 à 8, de formule générale Id 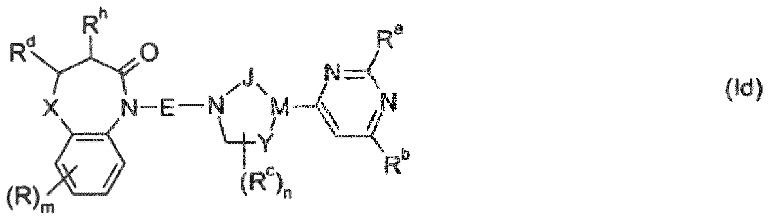 dans laquelle n, X, E, J, M, R, R^{a}, R^{b}, R^{c}, R^{d} et R^{h} présentent les significations indiquées précédemment et m représente 0, 1, 2 ou 3.

13. Composés selon l'une quelconque des revendications 1 à 8, de formule générale le 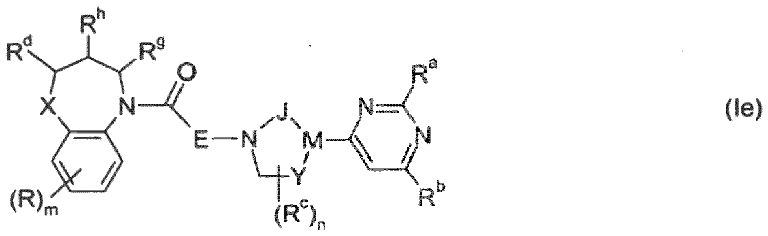 dans laquelle n, X, E, J, M, R, R^{a}, R^{b}, R^{c}, R^{d}, R^{g} et R^{h} présentent les significations indiquées précédemment et m représente 0, 1, 2 ou 3.

14. Agent pharmaceutique, contenant au moins un agent actif choisi parmi les composés de formule I et les sels d'addition acide physiologiquement compatibles des composés I selon l'une quelconque des revendications 1 à 13, éventuellement accompagné de véhicules et/ou d'adjuvants physiologiquement acceptables.

15. Utilisation d'agents actifs choisis parmi les composés de formule I et les sels d'addition acide physiologiquement compatibles des composés I selon l'une quelconque des revendications 1 à 13, pour la fabrication d'un agent pharmaceutique pour le traitement de maladies et de troubles choisis parmi les troubles et les maladies du système nerveux central et les dysfonctionnements rénaux, les troubles affectifs, les troubles névrotiques, les troubles liés au stress et somatoformes et les psychoses, notamment la schizophrénie et la dépression, ainsi que les dysfonctionnements rénaux, notamment les dysfonctionnements rénaux causés par le diabète sucré, qui répondent à l'influence des antagonistes ou agonistes des récepteurs D₃ de dopamine.

16. Utilisation selon la revendication 15 pour la fabrication d'un agent pharmaceutique pour le traitement de maladies du système nerveux central.

17. Utilisation selon la revendication 16 pour la fabrication d'un agent pharmaceutique pour le traitement de la schizophrénie et/ou de la dépression.
